# FASCICULE DE BREVET EUROPEEN

(11) **EP 1 275 641 B1**
(45) Date de publication et mention de la délivrance du brevet: **12.01.2005**
(21) Numéro de dépôt: 02291747.0
(22) Date de dépôt: 11.07.2002
(51) Int. Cl.: C07C 311/19, C07D 295/08, C07D 413/04, C07D 417/04, A61K 31/18, A61K 31/40, A61K 31/445, A61K 31/495, A61K 31/535

(54) **Dérivés de benzène sulfonamide, leur procédé de préparation et les compositions pharmaceutiques qui les contiennent**
Benzolsulfonamidderivate, Verfahren zu ihrer Herstellung und diese enthaltende pharmazeutische Zusammensetzungen
Benzenesulfonamide derivatives, process for their preparation and pharmaceutical compositions containing them

(30) Priorité: 13.07.2001 FR 0109339
(43) Date de publication de la demande: 15.01.2003
(73) Titulaire: Les Laboratoires Servier, 92415 Courbevoie Cedex (FR)
(72) Inventeur: Lavielle, Gilbert, 78170 La Celle Saint Cloud (FR); Dubuffet, Thierry, 76210 Bolbec (FR); Cimetiere, Bernard, 75020 Paris (FR); Verbeuren, Tony, 78540 Vernouillet (FR); Simonet, Serge, 78700 Conflans Sainte Honorine (FR); Vayssettes-Courchay, Christine, 91430 Igny (FR)

(56) Documents cités:
- EP-A- 0 398 326
- EP-A- 0 542 136
- WO-A-94/06761

## Description

La présente invention concerne des nouveaux dérivés de benzènesulfonamide, leur procédé de préparation et les compositions pharmaceutiques qui les contiennent.

Des composés possédant un enchaînement benzènesulfonamide ont été décrits dans la demande EP 864561 pour leur caractère donneur de NO et antagonistes des récepteurs au thromboxane A₂ (TXA₂), ainsi que dans les demandes EP 648741 ou WO 9406761 pour leurs seules propriétés antagonistes des récepteurs du TXA₂.

Les composés de la présente invention possèdent une structure originale qui leur confère un caractère antagoniste des récepteurs du TXA₂ et antagoniste des récepteurs sérotoninergiques 5HT₂.

L'agrégation plaquettaire et les vasospasmes jouent un rôle essentiel dans l'étiologie et le développement des maladies cardiovasculaires athéro-thrombotiques. Le TXA₂, métabolite de l'acide arachidonique, et la sérotonine (5HT), neurotransmetteur, sont tous deux de puissants agents vasoconstricteurs, et peuvent induire ou renforcer l'activation des plaquettes, conduisant à leur agrégation. Les actions vasoconstrictrices et proagrégantes du TXA₂ se font par l'intermédiaire de récepteurs membranaires appelé TP-récepteurs (Medicinal Research Reviews, 1991, 11, 5, p.503) alors que celles de la sérotonine se font par l'intermédiaire des récepteurs 5HT₁ ou 5HT₂ (T.I.P.S., 1991, 121, p.223). Les stratégies de recherche mises en oeuvre afin de trouver des agents qui bloquent la production et/ou l'activation du TXA₂ ont conduit au développement d'antagonistes sélectifs des récepteurs TP, d'inhibiteurs de TXA₂-synthase, ou d'agents mixtes possédant les deux propriétés (Medicinal Research Reviews, ibd., T.I.P.S., 1991, 121, 158). La sérotonine agit, tout comme le TXA₂, en stimulant les plaquettes et les constrictions vasculaires, et son activité se trouve renforcée dans les maladies athéro-thrombotiques.

La conception de composés s'opposant à la fois au processus faisant intervenir le thromboxane et à celui faisant intervenir la sérotonine, est d'une grande utilité pour les cliniciens. De tels produits présentent l'avantage d'offrir une protection plus complète, à la fois contre l'activation des plaquettes et contre les vasospasmes. Ils pourront donc être utiles pour le traitement des pathologies liées à une activité exagérée de TXA₂ et de 5-HT en particulier dans le traitement des maladies cardiovasculaires athéro-thrombotiques telles que l'infarctus du myocarde, l'angine de poitrine, les accidents vasculaires cérébraux, la maladie de Raynaud, ou encore l'asthme, les bronchospasmes, mais aussi la migraine et les maladies veineuses.

La présente invention concerne les composés de formule (I) : 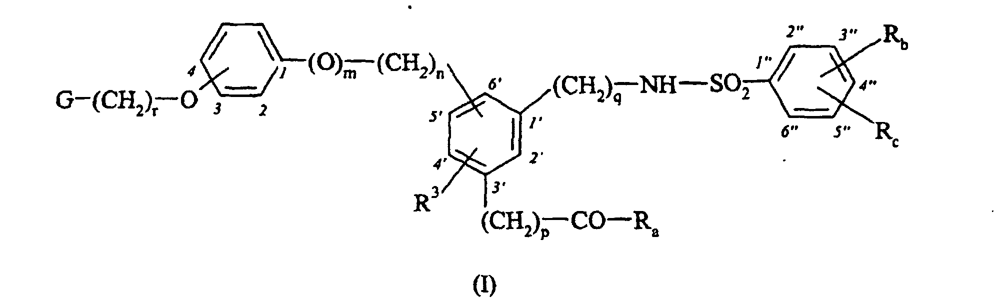 dans laquelle :
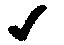 G représente un groupement tel que : 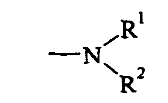 où R¹ et R² représentent indépendamment un atome d'hydrogène, un groupement alkyle, cycloalkyle, aryle éventuellement substitué, arylalkyle éventuellement substitué, cycloalkyle, cycloalkylalkyle, hétéroaryle éventuellement substitué, ou hétéroarylalkyle éventuellement substitué,
   ou bien,
   R¹ et R² forment ensemble avec l'atome d'azote un groupement hétérocycloalkyle de formule 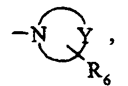 de 5 à 7 chaînons dans lequel Y représente un atome d'azote, d'oxygène ou un groupement CH₂ et R₆ représente un atome d'hydrogène, un groupement alkyle, cycloalkyle, cycloalkylalkyle, aryle éventuellement substitué, arylalkyle éventuellement substitué, arylcarbonyle éventuellement substitué, arylcarbonylalkyle éventuellement substitué, diarylalkyle éventuellement substitué, diarylalkényle éventuellement substitué, hétéroaryle éventuellement substitué, hétéroarylalkyle éventuellement substitué, hétéroarylcarbonyle éventuellement substitué, ou hétéroarylcarbonylalkyle éventuellement substitué,
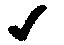 R³ représente un atome d'hydrogène, groupement alkyle, ou phényle éventuellement substitué,
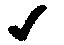 Rₐ représente un groupement hydroxy, alkoxy, aryloxy éventuellement substitué, arylalkyloxy éventuellement substitué, amino, alkylamino, dialkylamino, arylamino éventuellement substitué, arylalkylamino éventuellement substitué,
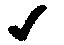 R_{b} et R_{c}, identiques ou différents, représentent un atome d'hydrogène, un atome d'halogène, un groupement alkyle, un groupement alkoxy, un groupement hydroxy ou un groupement trihalogénoalkyle,
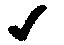 m est un entier compris inclusivement entre 0 et 1,
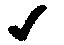 n et q sont des entiers identiques ou différents compris inclusivement entre 0 et 6,
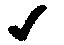 p et r sont des entiers identiques ou différents compris inclusivement entre 1 et 6,
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkoxy désigne un groupement alkyle-oxy, contenant de 1 à 6 atomes de carbone, linéaire ou ramifié,
- le terme trihalogénoalkyle désigne un chaîne carbonée, contenant de 1 à 3 atomes de carbone et de 1 à 3, identiques ou différents, atomes d'halogène,
- le terme alkényle désigne une chaîne de 2 à 6 atomes de carbone contenant de 1 à 3 doubles liaisons,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement monocyclique aromatique, ou bicyclique dans lequel au moins un des cycles est aromatique, contenant 5 à 11 chaînons, et 1 à 5 hétéroatomes choisis parmi azote, oxygène et soufre,
- les termes diarylalkyle et diarylalkényle désignent respectivement des groupements alkyle et alkényle tels que définis précédemment, substitués par deux groupements aryles, identiques ou différents, tels que définis précédemment,
- le terme "substitué" associé aux expressions phényle, aryle, arylalkyle, arylcarbonyle, arylcarbonylalkyle, diarylalkyle, diarylalkényle, hétéroaryle, hétéroarylalkyle, hétéroarylcarbonyle, hétéroarylcarbonylalkyle, arylamino et arylalkylamino signifie que les groupements concernés sont substitués sur la partie aromatique par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que les groupements hétéroaryle et hétéroarylalkyle peuvent être en plus substitués par un groupement oxo sur la partie non-aromatique de l'hétéroaryle.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les composés préférés de formule (I) sont ceux pour lesquels, pris ensemble ou séparément, la valeur de p est égale à 2, la valeur de q est égale à 2, le substituant R³ représente un atome d'hydrogène, le substituant Rₐ représente un groupement hydroxy, le substituant R_{b} représente un atome d'halogène et le substituant R_{c} représente un atome d'hydrogène.

Un aspect particulièrement avantageux de l'invention concerne les composés de formule (I) pour lesquels p et q valent chacun 2, Rₐ représente un groupement hydroxy, R³ et R_{c} représentent chacun un atome d'hydrogène, R_{b} représente un atome d'halogène, et G représente un groupement amino, dialkylamino, arylalkylamino, ou un groupement hétérocycloalkyle de formule 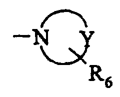 dans laquelle Y représente un atome d'azote, d'oxygène, ou un groupement CH₂ et R₆ est choisi parmi un atome d'hydrogène, les groupements aryle éventuellement substitué et hétéroaryle éventuellement substitué.

Dans les composés préférés de formule (I), G représente un groupement dialkylamino.

D'autres composés préférés de formule (I) sont ceux pour lesquels G représente un groupement hétérocycloalkyle substitué 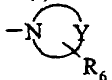 dans lequel :
- 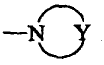 représente un groupement à 5 ou 6 chaînons tels que : pyrolyle, morpholino, pipéridyle ou pipérazinyle,
- et R₆ substitué sur un atome de carbone ou d'azote de l'hétérocycloalkyle, représente un atome d'hydrogène, un substituant phényle éventuellement substitué par un atome d'halogène, ou un groupement hétéroaryle à 9 chaînons comportant un à deux hétéroatomes choisis parmi l'atome d'azote, d'oxygène et de soufre, et éventuellement substitué par un atome d'halogène.

Les composés préférés de formule (I) sont ceux pour lesquels le groupement hétéroaryle éventuellement substitué représente un groupement benzisoxazolyle éventuellement substitué par un atome d'halogène ou benzisothiazolyle éventuellement substitué par un atome d'halogène.

De manière avantageuse, l'invention concerne les composés de formule (I) pour lesquels pris ensemble ou séparément, le substituant G-(CH₂)ᵣ-O- est relié à un des deux atomes de carbone 2 ou 4 du groupement phényle, le substituant 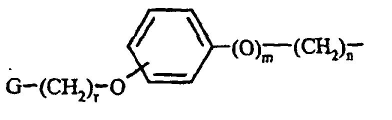 est relié à l'atome de carbone 5' du groupement phényle, et R_{b} est relié à l'atome 4" du groupement phényle.

Parmi les composés préférés de l'invention, on peut citer :
- l'acide 3-[3-(2-{[(4-chlorophényl)sulfonyl]amino}éthyl)-5-(2-{2-[3-(diméthylamino) propoxy]phényl}éthyl)phényl]propanoïque
- l'acide 3-[3-(2-{[(4-chlorophényl)sulfonyl]amino}éthyl)-5-(2-{2-[2-(1-pyrrolidinyl) éthoxy]phényl}éthyl)phényl]propanoïque
- l'acide 3-[3-(2-{[(4-chlorophényl)sulfonyl]anlino}éthyl)-5-(2-{2-[2-(4-morpholinyl) éthoxy]phényl}éthyl)phényl]propanoïque.

La présente invention concerne également le procédé de préparation des composés de formule (I), caractérisé en ce que :
① lorsque les composés de formule (I) que l'on souhaite obtenir sont tels que m=0 et n différent de 1, l'on utilise comme produit de départ un composé de formule (II) : 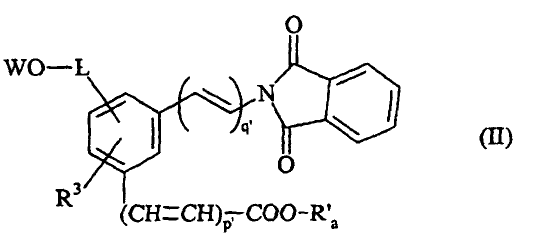 dans laquelle R³ a la même signification que dans la formule (I), R'ₐ représente un groupement alkyle (C₁-C₆) linéaire ou ramifié, WO-L représente un groupement de formule : 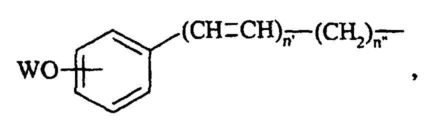 avec W représentant un atome d'hydrogène ou un groupement benzyle, p' et q' sont des entiers compris entre 0 et 3, n'+n"= un entier compris entre 0 et 3 qui lorsque n'vaut 0 alors n''vaut 0, 2 ou 3,
   - qui est réduit catalytiquement pour conduire après débenzylation, lorsque W représente un groupement benzyle, au composé de formule (III) : 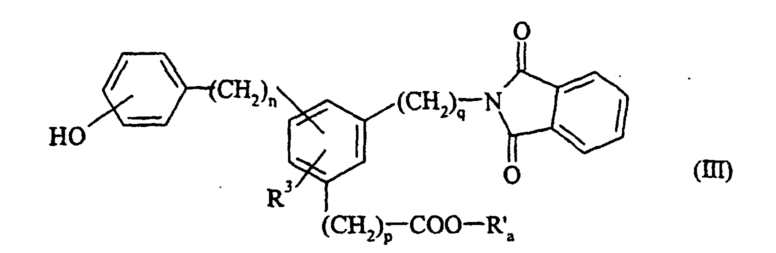 dans laquelle R³, n, p et q ont là même signification que dans la formule (I), R'ₐ représente un groupement alkyle (C₁-C₆) linéaire ou ramifié,
   - qui se condense en milieu basique avec un halogénure de formule : G-(CH₂)ᵣ-X, dans laquelle X représente un atome d'halogène pour conduire au composé de formule (IV) : 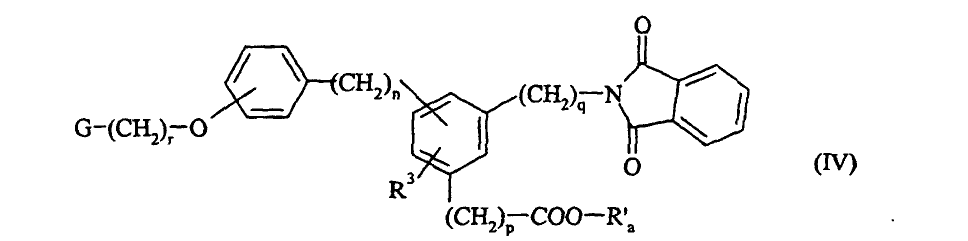 dans laquelle G, R³, n, p, q et r ont la même signification que dans la formule (I), R'ₐ représente un groupement alkyle (C₁-C₆) linéaire ou ramifié,
   - qui est déprotégé par clivage du groupement phtalimido en présence d'hydrazine pour conduire à l'amine correspondante qui, à son tour, réagit avec un halogénure de benzène sulfonyle éventuellement substitué de formule : 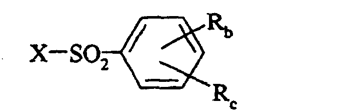 dans laquelle R_{b} et R_{c} ont la même signification que dans la formule (I) et X représente un atome d'halogène,
      pour donner le composé de formule (I/a), cas particulier des composés (I): 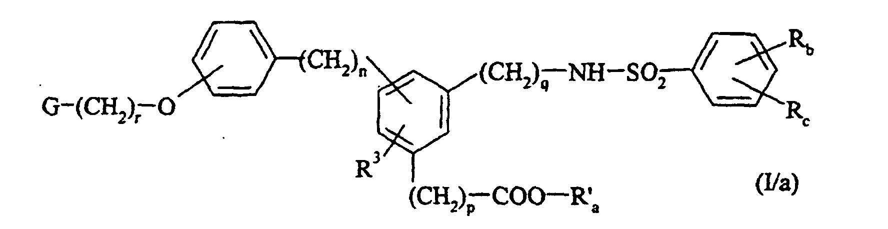 dans laquelle G, R_{b}, R_{c}, R³, r, n, p et q ont la même signification que dans la formule (I), R'ₐ représente un groupement alkyle (C₁-C₆) linéaire ou ramifié,
② lorsque les composés de formule (I) que l'on souhaite obtenir sont tels que m=1, ou m=0 avec n=1, l'on utilise comme produit de départ un composé de formule (II/a): 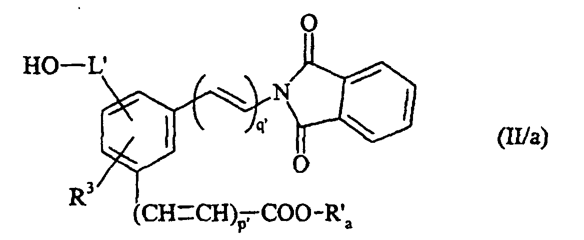 dans laquelle R³ a la même signification que dans la formule (I), R'ₐ représente un groupement alkyle (C₁-C₆) linéaire ou ramifié, HO-L' représente un groupement de formule : HO-(CH₂)ₙ-, q', p' sont des nombres entiers compris entre 0 et 3, et n est un nombre entier compris entre 0 et 6,
   - qui est réduit catalytiquement et halogéné pour conduire au composé (V) : 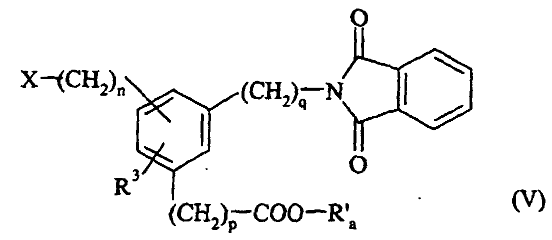 dans laquelle R³, n, p et q ont la même signification que dans la formule (I), R'ₐ représente un groupement alkyle (C₁-C₆) linéaire ou ramifié, X représente un atome d'halogène,
      composé de formule (V) qui subit :
   - **soit**, lorsque m=1, l'attaque nucléophile d'un composé de formule : 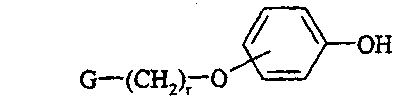 pour conduire au composé de formule (VI) : 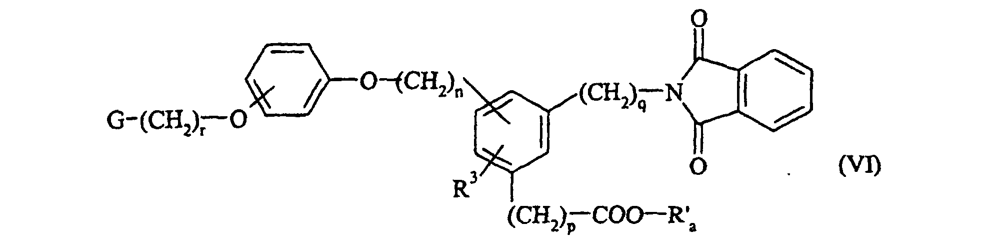 dans laquelle G, R³, n, p, q et r ont la même signification que dans la formule (I), R'ₐ représente un groupement alkyle (C₁-C₆) linéaire ou ramifié,
   - qui est déprotégé par clivage du groupement phtalimido en présence d'hydrazine pour conduire à l'amine correspondante qui, à son tour, réagit avec un halogénure de benzène sulfonyle éventuellement substitué de formule : 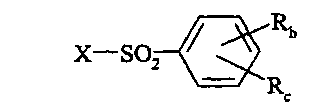 dans laquelle X représente un atome d'halogène, pour donner le composé de formule (I/b), cas particulier des composés de formule (I) : 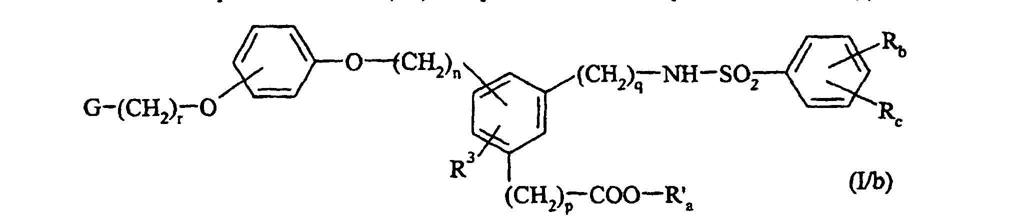 dans laquelle G, R_{b}, R_{c}, R³, n, p, q et r ont la même signification que dans la formule (I), R'ₐ représente un groupement alkyle (C₁-C₆) linéaire ou ramifié,
   - soit, lorsque m=0 et n=1, l'action d'un composé de formule 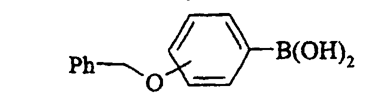 pour conduire après débenzylation au composé de formule (VII) : 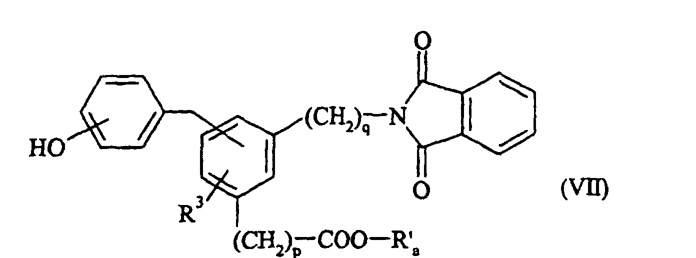 dans laquelle R³, p et q ont la même signification que dans la formule (I), R'ₐ représente un groupement alkyle (C₁-C₆) linéaire ou ramifié,
   - qui se condense en milieu basique avec un halogénure de formule G-(CH₂)ᵣ-X, dans laquelle X représente un atome d'halogène pour conduire au composé de formule (VIII) : 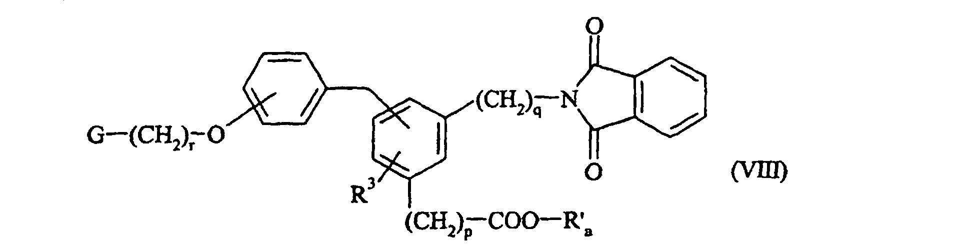 dans laquelle G, R³, p, q et r ont la même signification que dans la formule (I), R'ₐ représente un groupement alkyle (C₁-C₆) linéaire ou ramifié,
   - qui est déprotégé par clivage du groupement phtalimido en présence d'hydrazine pour conduire à l'amine correspondante qui, à son tour, réagit avec un halogénure de benzène sulfonyle éventuellement substitué de formule : 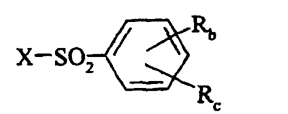 pour donner le composé de formule (I/c), cas particulier des composés (I): 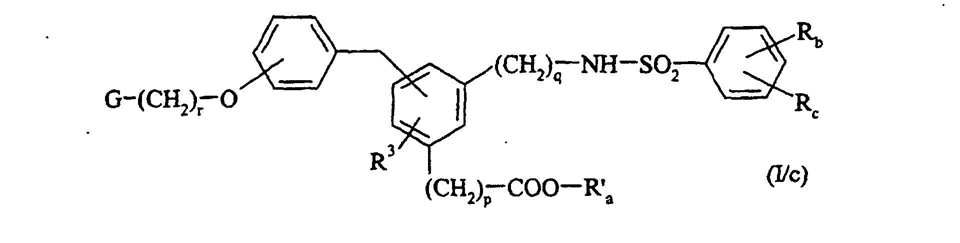 dans laquelle G, R_{b}, R_{c}, R³, p, q et r ont la même signification que dans la formule (I), R'ₐ représente un groupement alkyle (C₁-C₆) linéaire ou ramifié,
      composés de formule (I/a), (I/b) ou (I/c), qui peuvent être soumis à une hydrolyse de la fonction ester, en milieu basique ou acide selon les groupements réactifs présents sur la molécule, pour conduire au composé de formule (I/d) : 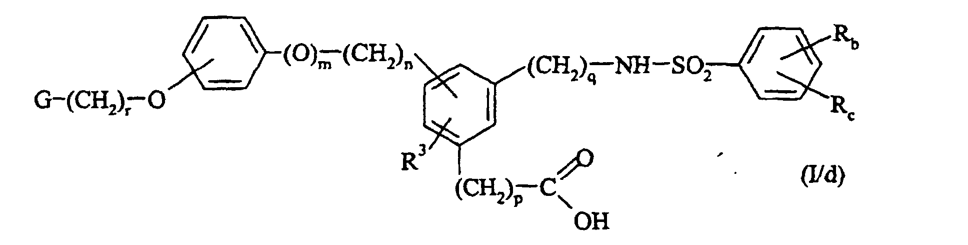 cas particulier des composés de formule (I) G, R_{b}, R_{c}, R³, m, n, p, q et r sont tels que définis dans la formule (I),
   composés (I/a), (I/b), (I/c) et (I/d) formant la totalité des composés de formule (I), et :
   - qui peuvent être, le cas échéant, purifiés selon une technique classique de purification,
   - dont on sépare, le cas échéant, les stéréoisomères selon une technique classique de séparation,
   - que l'on transforme, si on le souhaite, en leurs sels d'addition à un acide ou à une base pharmaceutiquement acceptable,
étant entendu qu'à tout moment jugé opportun au cours du procédé précédemment décrit, la fonction acide carboxylique peut être estérifiée ou bien la fonction ester carboxylique peut être hydrolysée en acide correspondant, ce dernier pouvant être à nouveau transformé en un autre ester pour les besoins de la synthèse.

La présente invention a également pour objet les compositions pharmaceutiques renfermant comme principe actif au moins un composé de formule (I) seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques et spectrométriques usuelles.

Les produits de départ utilisés sont des produits connus ou préparés selon des modes opératoires connus.

### Préparation A : (2E)-3-{3-[(E)-2-(1,3-Dioxo-1,3-dihydro-2H-isoindol-2-yl)éthényl]-5-[(E)-2-(2-hydroxyphényl)éthényl]phényl}-2-propénoate de tert-butyle

### Stade a : 3,5-Dibromobenzaldéhyde

A 0,222 moles de 1,3,5-tribromobenzène dans 1,8 litres d'éther éthylique à -80°C sont ajoutées goutte à goutte 0,222 moles d'une solution de *n*-butyllithium dans l'hexane. Le milieu réactionnel est agité à -80°C pendant une heure. Une solution de 0,222 moles de *N,N-*diméthylformamide dans 50 ml d'éther éthylique est additionnée lentement. La température est gardée à -80°C pendant une heure avant d'être ramenée à température ambiante. L'agitation est poursuivie pendant une nuit. 450 ml d'acide chlorhydrique 1 N sont additionnés. Le milieu réactionnel est extrait à l'éther. Les phases organiques sont rassemblées et séchées sur du sulfate de magnésium. Après filtration et évaporation des solvants, le produit brut est purifié par chromatographie sur colonne de silice (éluant : cyclohexane/acétate d'éthyle : 95/5).

### Stade b : 2-[(E)-2-(3,5-Dibromophényl)éthényl]phénol

A 0,237 moles de bromure de (2-hydroxybenzyl)(triphényl)phosphonium dans 1,2 litres de THF à +5°C sont ajoutées 0,474 moles d'une solution de *n*-butyllithium dans l'hexane. Le milieu réactionnel est gardé pendant une heure à +5°C puis 0,21 moles du composé obtenu à l'étape précédente en solution dans 360 ml de THF sont ajoutées goutte à goutte. Le milieu réactionnel est ramené lentement à température ambiante puis hydrolysé et extrait à l'éther éthylique. Les phases organiques sont collectées et séchées sur sulfate de magnésium. Après filtration et évaporation des solvants, le produit brut est purifié par chromatographie sur colonne de silice (éluant : cyclohexane/acétate d'éthyle : 95/5).

### Stade c : 3-Bromo-5-[(E)-2-(2-hydroxyphényl)éthényl]benzaldéhyde

A 0,172 moles du composé obtenu à l'étape précédente dans 3 litres d'éther éthylique à - 80°C sont ajoutées lentement 0,378 moles d'une solution de *n*-butyllithium dans l'hexane. Le milieu réactionnel est maintenu à -80°C pendant une heure puis 0,172 moles de *N*,*N*-diméthylformamide en solution dans 100 ml d'éther sont additionnées lentement. Le milieu réactionnel est gardé à cette température pendant une heure puis est amené à +10°C pour être hydrolysé par une solution d'acide chlorhydrique 1 N. Le milieu est extrait par de l'éther éthylique, les phases organiques sont collectées et séchées sur sulfate de magnésium. Après filtration et évaporation des solvants, le produit brut est purifié par chromatographie sur silice (éluant : cyclohexane/acétate d'éthyle : 90/10).

### Stade d : (2E)-3-{3-Bromo-5-[(E)-2-(2-hydroxyphényl)éthényl]phényl}-2-propénoate de tert-butyle

A 0,15 moles du composé obtenu à l'étape précédente dans 2 litres de toluène sont additionnées 0,3 moles de triphényl phosphoranylidène acétate de *tert*-butyle. Le milieu réactionnel est porté au reflux pendant 2 heures. Les solvants sont évaporés et le produit brut est purifié par chromatographie sur colonne de silice (éluant : cyclohexane/acétate d'éthyle : 85/15).

### Stade e : (2E)-3-{3-[(E)-2-(1,3-Dioxo-1,3-dihydro-2H-isoindol-2-yl)éthényl]-5-[(E)-2-(2-hydroxyphényl)éthényl]phényl}-2-propénoate de tert-butyle

A 2,5 litres de *N*,*N*-diméthylformamide et 10 ml d'eau sont ajoutées successivement 0,1607 moles du composé obtenu au stade précédent, 0,016 moles d'acétate de palladium, 0,032 moles de tris(2-méthylphényl)phosphine, 0,402 moles de diisopropylamine, 0,1607 moles de vinylphtalimide. Le milieu réactionnel est porté au reflux pendant 3 heures puis ramené à température ambiante. Après hydrolyse, le milieu est amené à pH : 4 par une solution diluée d'acide chlorhydrique puis extrait par du dichlorométhane. Les phases organiques sont collectées et séchées sur sulfate de magnésium. Après filtration et évaporation des solvants, le produit brut est purifié par chromatographie sur silice (éluant : cyclohexane/acétate d'éthyle : 70/30).

### Préparation B : (2E)-3-[3-[(E)-2-(1,3-Dioxo-1,3-dibydro-2H-isoindol-2-yl)éthényl]-5-(hydroxyméthyl)phényl]-2-propénoate de tert-butyle

### Stade a : 3,5-Dibromobenzaldéhyde

Le produit est obtenu selon le même procédé décrit dans la Préparation A, Stade a.

### Stade b : (3,5-Dibromophényl)méthanol

A 0,3 moles du composé obtenu au stade précédent dans 650 ml de méthanol et 200 ml de THF sont ajoutées par portions 0,506 moles de borohydrure de sodium en maintenant la température à +30°C. Après 4 heures d'agitation, 300 ml d'une solution saturée de NaHCO₃ sont ajoutées. Les solvants sont évaporés. Le solide obtenu est filtré, lavé à l'eau et séché.

### Stade c : 3-Bromo-5-(hydroxyméthyl)benzaldéhyde

A 0,285 moles du composé obtenu au stade précédent dans 2,3 litres d'éther à -80°C sont ajoutées 0,628 moles de *n*-butyllithium. Le milieu réactionnel est maintenu à cette température pendant 4h30. 22 ml de DMF en solution dans 100 ml d'éther sont ajoutés à -80°C. Le milieu est gardé à -80°C pendant 1 heure avant d'être autorisé à remonter jusqu'à une température de +10°C. Un solution d'acide chlorhydrique 1 N est additionnée jusqu'à pH acide. Le milieu est décanté, extrait à l'éther éthylique et séché sur sulfate de magnésium. Le produit brut est purifié par chromatographie (éluant : cyclohexane/acétate d'éthyle : 80/20).

### Stade d : (2E)-3-[3-bromo-5-(hydroxyméthyl)phényl]-2-propénoate de tert-butyle

A 0,175 moles du composé obtenu au stade précédent dans 1,65 litres de toluène sont ajoutées 0,35 moles de triphénylphosphoranylidène acétate de *tert*-butyle. Le milieu réactionnel est porté à reflux pendant 3 heures. Les solvants sont évaporés. Le produit brut est repris dans l'éther iso-propylique et porté à reflux 1 heure. Après un refroidissement lent, le précipité est filtré. Le filtrat est évaporé et le produit brut est purifié par chromatographie sur colonne de silice (éluant : cyclohexane/acétate d'éthyle : 85/15).

### Stade e : (2E)-3-[3-[(E)-2-(1,3-dioxo-1,3-dihydro-2H-isoindol-2-yl)éthényl]-5-(hydroxyméthyl)phényl]-2-propénoate de tert-butyle

Le mode opératoire est identique à celui utilisé dans la Préparation A, Stade e.

### Préparation C : (2E)-3-{3-{(2E)-3-[2-(Benzyloxy)phényl]-2-propènyl}-5-[(E)-2-(1,3-dioxo-1,3-dihydro-2H-isoindol-2-yl)éthényl]phényl}-2-propénoate de tert-butyle

### Stade a : Bromure de (3,5-dibromobenzyl)(triphényl)phosphonium

A 0,20 moles du composé obtenu dans la Préparation A, Stade a, dans 800 ml d'acétonitrile, sont ajoutées par portions 0,20 moles de bromohydrate de triphényl phosphine. Le milieu est porté au reflux pendant 24 heures puis refroidi. Le solvant est ensuite évaporé, le précipité est filtré puis séché.

### Stade b : [2-(Benzyloxy)phényl]acétaldéhyde

A 0,08 moles de [2(2-benzyloxy)phényl]éthanol dans 500 ml de tétrahydrofurane, sont ajoutées 0,104 moles d'hydroxy-1-oxo-benzo[*d*][1,2]iodoxol-3-one. Le milieu réactionnel est chauffé à reflux pendant 2 heures puis refroidi. Le précipité est filtré et le filtrat évaporé.

### Stade c : 1-{(2E)-3-[2-(Benzyloxy)phényl]-2-propènyl}-3,5-dibromobenzène

A 0,13 moles du composé obtenu au Stade a, dans 800 ml de tétrahydrofurane sont ajoutées par portions 0,13 moles de terbutylate de potassium. Le milieu réactionnel est agité 30 minutes à température ambiante puis sont ajoutées 0,066 moles du composé obtenu au stade précédent. Après 12 heures d'agitation sont additionnées 150 ml d'eau puis on extrait par du dichlorométhane. Les phases organiques sont collectées et séchées sur du sulfate de magnésium. Le produit est purifié par chromatographie sur silice (éluant : cyclohexane/dichlorométhane : 80/20).

### Stade d : (2E)-3-(3-{(2E)-3-[2-(Benzyloxy)phényl]-2-propènyl}-5-bromophényl)-2-propènoate de tert-butyle

Le mode opératoire est identique à celui utilisé dans la Préparation A, Stade c et d.

### Stade e : (2E)-3-{3-{(2E)-3-[2-(Benzyloxy)phényl]-2-propènyl}-5-[(E)-2-(1,3-dioxo-1,3-dihydro-2H-isoindol-2-yl)éthényl]phényl}-2-propènoate de tert-butyle

Le mode opératoire est identique à celui utilisé dans la Préparation A, Stade e.

### EXEMPLE 1 : Acide 3-[3-(2-{[(4-chlorophényl)sulfonyl]amino}éthyl)-5-(2-{2-[2-(diméthylamino)éthoxy]phényl}éthyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

A 0,048 moles du composé obtenu dans la Préparation A, Stade e, dissout avec 720 ml d'éthanol, sont ajoutées 5 mmol de chlorotris(triphénylphosphine)rhodium. Le milieu réactionnel est porté à +50°C sous une pression de 6 atm d'hydrogène pendant 3 jours. Après retour à température ambiante, les solvants sont évaporés et le produit brut est purifié par chromatographie sur colonne de silice (éluant : cyclohexane/acétate d'éthyle : 80/20).

### Stade b : 3-{3-(2-{2-[2-(Diméthylamino)éthoxy]phényl}éthyl)-5-[2-(1,3-dioxo-1,3-dihydro-2H-isoindol-2-yl)éthyl]phényl}propanoate de tert-butyle

A 0,01 mole d'hydrure de sodium, préalablement lavé au pentane, mis en suspension dans 100 ml de toluène sont additionnées lentement 0,01 mole du composé au stade précédent en solution dans 100 ml de toluène. Le milieu réactionnel est porté au reflux pendant 30 minutes puis 0,013 moles de 2-chloro-*N,N*-diméthyléthanamine en solution dans 40 ml de toluène sont ajoutées lentement. Le reflux est maintenu pendant 7 heures. Après retour à température ambiante, le milieu réactionnel est hydrolysé suivi d'une extraction à l'éther éthylique. Les phases organiques sont collectées, lavées par une solution 1 N de soude et séchées par du sulfate de magnésium.

### Stade c : 3-[3-(2-Aminoéthyl)-5-(2-{2-[2-(diméthylamino)éthoxy]phényl}éthyl) phényl]propanoate de tert-butyle

A 0,01 mole du composé obtenu au stade précédent dans 27 ml de méthanol et 4,5 ml d'eau sont ajoutées 0,0115 moles de monohydrate d'hydrazine. Le milieu est porté au reflux pendant 2h30 puis refroidi. Après addition de 91 ml d'une solution 1 N de carbonate de potassium et 75 ml de dichlorométhane, décantation et extraction une fois par du dichlorométhane, les solvants sont évaporés. Le produit brut est purifié par une chromatographie sur colonne de silice (éluant : dichlorométhane/méthanol/ammoniaque : 95/5/0,5).

### Stade d : 3-[3-(2-{[(4-Chlorophényl)sulfonyl]amino}éthyl)-5-(2-{2-[2-(diméthylamino)éthoxy]phényl}éthyl)phényl]propanoate de tert-butyle

A 0,005 moles du composé obtenu au stade précédent dans 50 ml de dichlorométhane sont additionnées 0,005 moles de triéthylamine. Le milieu réactionnel est refroidi à +5°C. 0,005 moles de chlorure de 4-chlorobenzènesulfonyle sont ajoutées. Le milieu est ramené à température ambiante puis agité 45 minutes avant d'être hydrolysé. Après extraction par du dichlorométhane et séchage sur sulfate de magnésium, les solvants sont évaporés. Le produit brut est utilisé sans autre purification dans l'étape suivante.

### Stade e : Acide 3-[3-(2-{[(4-chlorophényl)sulfonyl]amino}éthyl)-5-(2-{2-[2-(diméthylamino)éthoxyl]phényl}éthyl)phényl]propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit est extrait à l'acétate d'éthyle. Les phases organiques sont collectées et séchées sur du sulfate de magnésium. Le solvant est évaporé.

| *Microanalyse élémentaire :* | | | | |
|---|---|---|---|---|
| | C | H | N | S |
| % calculé | 62,30 | 6,31 | 5,01 | 5,73 |
| % trouvé | 61,92 | 6,43 | 4,85 | 5,38 |

### EXEMPLE 2 : Acide 3-[3-(2-{[(4-chlorophényl)sulfonyl]amino}éthyl)-5-(2-{2-[3-(diméthylamino)propoxy]phényl}éthyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-(2-{2-[3-(Diméthylamino)propoxy]phényl}éthyl)-5-[2-(1,3-dioxo-1,3-dihydro-2H-isoindol-2-yl)éthyl]phényl}propanoate de tert-butyle

Le produit attendu est obtenu selon le procédé décrit dans l'Exemple 1, Stade b en remplaçant le 2-chloro-*N,N*-diméthyléthanamine par le *N*-(3-chloropropyl)-*N,N*-diméthylamine.

### Stade c : 3-[3-(2-Aminoéthyl)-5-(2-{2-[3-(diméthylamino)propoxy]phényl}éthyl) phényl]propanoate de tert-butyle

Les conditions expérimentales pour obtenir le produit sont identiques à celles utilisées lors de l'Exemple 1, Stade c.

### Stade d : 3-(3-(2-{2-[3-(Diméthylamino)propoxy]phényl}éthyl)-5-{2-[(phénylsulfonyl)amino]éthyl}phényl)propanoate de tert-butyle

Les conditions expérimentales pour obtenir le produit sont identiques à celles utilisées lors de l'Exemple 1, Stade d.

### Stade e : Acide 3-[3-(2-{[(4-chlorophényl)sulfonyl]amino}éthyl)-5-(2-{2-[3-(diméthylamino)propoxy]phényl}éthyl)phényl]propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit est purifié par précipitions, filtration, lavage à l'eau, et enfin séché.

| *Microanalyse élémentaire :* | | | | | |
|---|---|---|---|---|---|
| | C | H | Cl | N | S |
| % calculé | 62,87 | 6,51 | 6,19 | 4,89 | 5,59 |
| % trouvé | 62,76 | 6,58 | 6,30 | 4,81 | 5,51 |

### EXEMPLE 3: Acide 3-[3-(2-{[(4-chlorophényl)sulfonyl]amino}éthyl)-5-(2-{2-[2-(1-pyrrolidinyl)éthoxy]phényl}éthyl)phényl]propanoïque

### Stade a : 3-{3-[2-(1,3-Dioxo-1,3-dihydro-2H-isoindol-2-yl)éthyl]-5-[2-(2hydroxyphényl)éthyl]phényl}propanoate de tert-butyle

Le procédé expérimental est identique à celui de l'Exemple 1, Stade a.

### Stade b : 3-[3-[2-(1,3-Dioxo-1,3-dihydro-2H-isoindol-2-yl)éthyl]-5-(2-{2-[2-(1-pyrrolidinyl)éthoxy]phényl}éthyl)phényl]propanoate de tert-butyle

Le produit attendu est obtenu selon le procédé décrit dans l'Exemple 1, Stade b en remplaçant le 2-chloro-*N,N*-diméthyléthanamine par le 1-(2-chloroéthyl)pyrrolidine.

### Stade c : 3-[3-(2-Aminoéthyl)-5-(2-{2-[2-(1-pyrrolidinyl)éthoxy]phényl}éthyl) phényl]propanoate de tert-butyle

Les conditions expérimentales pour obtenir le produit sont identiques à celles utilisées lors de l'Exemple 1, Stade c.

### Stade d : 3-[3-(2-{[(4-Chlorophényl)sulfonyl]amino}éthyl)-5-(2-{2-[2-(1-pyrrolidinyl)éthoxy]phényl}éthyl)phényl]propanoate de tert-butyle

Les conditions expérimentales pour obtenir le produit sont identiques à celles utilisées lors de l'Exemple 1, Stade d.

### Stade e : Acide 3-[3-(2-{[(4-chlorophényl)sulfonyl]amino}éthyl)-5-(2-{2-[2-(1-pyrrolidinyl)éthoxy]phényl}éthyl)phényl]propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit est extrait à l'acétate d'éthyle. Les phases organiques sont collectées et séchées sur sulfate de magnésium. Le solvant est évaporé. Le produit est purifié par chromatographie sur colonne de silice (éluant : dichlorométhane/méthanol/ammoniaque : 90/10/1).

| *Microanalyse élémentaire :* | | | | | |
|---|---|---|---|---|---|
| | C | H | Cl | N | S |
| % calculé | 63,63 | 6,37 | 6,06 | 4,79 | 5,48 |
| % trouvé | 63,30 | 6,47 | 6,30 | 4,83 | 5,23 |

### EXEMPLE 4 : Acide 3-[3-(2-{[(4-chlorophényl)sulfonyl]amino}éthyl)-5-(2-{2-[2-(4-morpholinyl)éthoxy]phényl}éthyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-[3-[2-(1,3-Dioxo-1,3-dihydro-2H-isoindol-2-yl)éthyl]-5-(2-{2-[2-(4-morpholinyl)éthoxy]phényl}éthyl)phényl]propanoate de tert-butyle

Le produit attendu est obtenu selon le procédé décrit dans l'Exemple 1, Stade b en remplaçant le 2-chloro-*N*,*N*-diméthyléthanamine par le 4-(2-chloroéthyl)morpholine.

### Stade c : 3-[3-(2-Aminoéthyl)-5-(2-{2-[2-(4-morpholinyl)éthoxy]phényl}éthyl) phényl]propanoate de tert-butyle

Les conditions expérimentales pour obtenir le produit sont identiques à celles utilisées lors de l'Exemple 1, Stade c.

### Stade d : 3-[3-(2-{[(4-chlorophényl)sulfonyl]amino}éthyl)-5-(2-{2-[2-(4-morpholinyl)éthoxy]phényl}éthyl)phényl]propanoate de tert-butyle

Les conditions expérimentales pour obtenir le produit sont identiques à celles utilisées lors de l'Exemple 1, Stade d.

### Stade e : Acide 3-[3-(2-{[(4-chlorophényl)sulfonyl]amino}éthyl)-5-(2-{2-[2-(4-morpholinyl)éthoxy]phényl}éthyl)phényl]propanoïque

Le produit attendu est obtenu selon le même procédé décrit dans l'Exemple 1, Stade e.

| *Microanalyse élémentaire :* | | | | | |
|---|---|---|---|---|---|
| | C | H | Cl | N | S |
| % calculé | 61,94 | 6,20 | 5,90 | 4,66 | 5,33 |
| % trouvé | 61,82 | 6,26 | 6,21 | 4,70 | 5,10 |

### EXEMPLE 5 : Acide 3-[3-(2-{[(4-chlorophényl)sulfonyl]amino}éthyl)-5-(2-{4-[2-(diméthylamino)éthoxy]phényl}éthyl)phényl]propanoïque

### Stade a : 3-{3-[2-(1,3-Dioxo-1,3-dihydro-2H-isoindol-2-yl)éthyl]-5-[2-(4-hydroxyphényl)éthyl]phényl}propanoate de tert-butyle

Le procédé expérimental est identique à celui de l'Exemple 1, Stade a, en utilisant comme réactif de Wittig lors de la Préparation A, Stade b le bromure de (4-hydroxybenzyl) (triphényl)phosphonium à la place du bromure de (2-hydroxybenzyl)(triphényl) phosphonium.

### Stade b : 3-{3-(2-{4-[2-(Diméthylamino)éthoxy]phényl}éthyl)-5-[2-(1,3-dioxo-1,3-dihydro-2H-isoindol-2-yl)éthyl]phényl}propanoate de tert-butyle

Le produit attendu est obtenu selon le procédé décrit dans l'Exemple 1, Stade b.

### Stade c : 3-[3-(2-Aminoéthyl)-5-(2-{4-[2-(diméthylamino)éthoxy]phényl}éthyl) phényl]propanoate de tert-butyle

Le conditions expérimentale pour obtenir le produit sont identiques à celles utilisées lors de l'Exemple 1, Stade c.

### Stade d : 3-[3-(2-{[(4-Chlorophényl)sulfonyl]amino}éthyl)-5-(2-{4-[2 (diméthylamino)éthoxy]phényl}éthyl)phényl]propanoate de tert-butyle

Le conditions expérimentale pour obtenir le produit sont identiques à celles utilisées lors de l'Exemple 1, Stade d.

### Stade e : Acide 3-[3-(2-{[(4-chlorophényl)sulfonyl]amino}éthyl)-5-(2-{4-[2-(diméthylamino)éthoxy]phényl}éthyl)phényl]propanoïque

Le produit attendu est obtenu selon la même méthode que celle décrite dans l'Exemple 1, Stade e, suivie d'une purification par chromatographie sur colonne de silice (dichlorométhane/méthanol/ammoniaque: 90/10/1).

| *Microanalyse élémentaire :* | | | | | |
|---|---|---|---|---|---|
| | C | H | Cl | N | S |
| % calculé | 62,30 | 6,31 | 6,34 | 5,01 | 5,73 |
| % trouvé | 62,02 | 6,14 | 6,08 | 5,03 | 5,36 |

### EXEMPLE 6 : Acide 3-{3-(2-{[(4-chlorophényl)sulfonyl]amino}éthyl)-5-[2-(2-{2-[4-(4-fluorophényl)-1-pipérazinyl]éthoxy}phényl)éthyl]phényl} propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(1,3-Dioxo-1,3-dihydro-2H-isoindol-2-yl)éthyl]-5-[2-(2-{2-[4-(4-fluorophényl)-1-pipérazinyl]éthoxy}phényl)éthyl]phényl}propanoate de tert-butyle

Le produit attendu est obtenu selon le procédé décrit dans l'Exemple 1, Stade b en remplaçant le 2-chloro-*N,N*-diméthyléthanamine par le 1-(2-chloroéthyl)-4-(4-fluorophényl)pipérazine.

### Stade c : 3-{3-(2-Aminoéthyl)-5-[2-(2-{2-[4-(4-fluorophényl)-1-pipérazinyl] éthoxy}phényl)éthyl]phényl}propanoate de tert-butyle

Les conditions expérimentales pour obtenir le produit sont identiques à celles utilisées lors de l'Exemple 1, Stade c.

### Stade d : 3-{3-(2-{[(4-Chlorophényl)sulfonyl]amino}éthyl)-5-[2-(2-{2-[4-(4-fluorophényl)-1-pipérazinyl]éthoxy}phényl)éthyl]phényl}propanoate de tert-butyle

Les conditions expérimentales pour obtenir le produit sont identiques à celles utilisées lors de l'Exemple 1, Stade d.

### Stade e : Acide 3-{3-(2-{[(4-chlorophényl)sulfonyl]amino}éthyl)-5-[2-(2-{2-[4-(4-fluorophényl)-1-pipérazinyl]éthoxy}phényl)éthyl]phényl}propanoïque

Le produit attendu est obtenu selon la même méthode que celle décrite dans l'Exemple 3, Stade e, à l'éluant près lors de la purification par chromatographie sur colonne de silice (dichlorométhane/méthanol/ammoniaque : 97/3/0,3).

| *Microanalyse élémentaire :* | | | | | |
|---|---|---|---|---|---|
| | C | H | Cl | N | S |
| % calculé | 64,01 | 5,95 | 5,11 | 6,05 | 4,62 |
| % trouvé | 63,58 | 6,17 | 5,42 | 5,95 | 4,31 |

### EXEMPLE 7: Acide 3-{3-(2-{[(4-chlorophényl)sulfonyl]amino}éthyl)-5-[2-(2-{3-[4-(4-fluorophényl)-1-pipérazinyl]propoxy}phényl)éthyl]phényl} propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(1,3-Dioxo-1,3-dihydro-2H-isoindol-2-yl)éthyl]-5-[2-(2-{3-[4-(4-fluorophényl)-1-pipérazinyl]propoxy}phényl)éthyl]phényl}propanoate de tert-butyle

Le produit attendu est obtenu selon le procédé décrit dans l'Exemple 1, Stade b en remplaçant le 2-chloro-*N,N*-diméthyléthanamine par le 1-(3-chloropropyl)-4-(4-fluorophényl)pipérazine.

### Stade c : 3-{3-(2-Aminoéthyl)-5-[2-(2-{3-[4-(4-fluorophényl)-1-pipérazinyl] propoxy}phényl)éthyl]phényl}propanoate de tert-butyle

Les conditions expérimentales pour obtenir le produit sont identiques à celles utilisées lors de l'Exemple 1, Stade c.

### Stade d : 3-{3-(2-{[(4-Chlorophényl)sulfonyl]amino}éthyl)-5-[2-(2-{3-[4-(4-fluorophényl)-1-pipérazinyl]propoxy}phényl)éthyl]phényl}propanoate de tert-butyle

Les conditions expérimentales pour obtenir le produit sont identiques à celles utilisées lors de l'Exemple 1, stade d.

### Stade e : Acide 3-{3-(2-{[(4-chlorophényl)sulfonyl]amino}éthyl)-5-[2-(2-{3-[4-(4-fluorophényl)-1-pipérazinyl]propoxy}phényl)éthyl]phényl}propanoïque

Le produit attendu est obtenu selon le même procédé décrit dans l'Exemple 1, Stade e.

| *Microanalyse élémentaire :* | | | | | |
|---|---|---|---|---|---|
| | C | H | Cl | N | S |
| % calculé | 64,44 | 6,12 | 5,01 | 5,93 | 4,53 |
| % trouvé | 63,49 | 6,00 | 5,09 | 5,84 | 4,13 |

### EXEMPLE 8 : Acide 3-[3-(2-{[(4-chlorophényl)sulfonyl]amino}éthyl)-5-(2-{2-[4-(diméthylamino)butoxy]phényl}éthyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-(2-{2-[4-(Diméthylamino)butoxy]phényl}éthyl)-5-[2-(1,3-dioxo-1,3-dihydro-2H-isoindol-2-yl)éthyl]phényl}propanoate de tert-butyle

Le produit attendu est obtenu selon le procédé décrit dans l'Exemple 1, Stade b en remplaçant le 2-chloro-*N,N*-diméthyléthanamine par le *N*-(4-chlorobutyl)-*N,N*-diméthylamine.

### Stade c : 3-[3-(2-Aminoéthyl)-5-(2-{2-[4-(diméthylamino)butoxy]phényl}éthyl) phényl]propanoate de tert-butyle

Les conditions expérimentales pour obtenir le produit sont identiques à celles utilisées lors de l'Exemple 1, Stade c.

### Stade d : 3-[3-(2-{[(4-Chlorophényl)sulfonyl]amino}éthyl)-5-(2-{2-[4-(diméthylamino)butoxy]phényl}éthyl)phényl]propanoate de tert-butyle

Les conditions expérimentales pour obtenir le produit sont identiques à celles utilisées lors de l'Exemple 1, Stade d.

### Stade e : Acide 3-[3-(2-{[(4-chlorophényl)sulfonyl]amino}éthyl)-5-(2-{2-[4-(diméthylamino)butoxy]phényl}éthyl)phényl]propanoïque

Le produit attendu est obtenu selon la même méthode que celle décrite dans l'Exemple 3, Stade e, à l'éluant près lors de la purification par chromatographie sur colonne de silice (dichlorométhane/méthanol/ammoniaque : 97/3/0,3).

| *Microanalyse élémentaire :* | | | | | |
|---|---|---|---|---|---|
| | C | H | Cl | N | S |
| % calculé | 63,41 | 6,69 | 6,04 | 4,77 | 5,46 |
| % trouvé | 63,38 | 6,96 | 6,12 | 4,74 | 5,21 |

### EXEMPLE 9 : Acide 3-{3-(2-{[(4-chlorophényl)sulfonyl]amino}éthyl)-5-[2-(2-{2-[4-(6-fluoro-1,2-benzisoxazol-3-yl)-1-pipéridinyl]éthoxy}phényl)éthyl] phényl}propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(1,3-Dioxo-1,3-dihydro-2H-isoindol-2-yl)éthyl]-5-[2-(2-{2-[4-(6-fluoro-1,2-benzisoxazol-3-yl)-1-pipéridinyl]éthoxy}phényl)éthyl]phényl} propanoate de tert-butyle

Le produit attendu est obtenu selon le procédé décrit dans l'Exemple 1, Stade b en remplaçant le 2-chloro-*N,N*-diméthyléthanamine par le 3-[1-(2-chloroéthyl)-4-pipéridinyl]-6-fluoro-1,2-benzisoxazole.

### Stade c : 3-{3-(2-Aminoéthyl)-5-[2-(2-{2-[4-(6-fluoro-1,2-benzisoxazol-3-yl)-1-pipéridinyl]éthoxy}phényl)éthyl]phényl}propanoate de tert-butyle

Les conditions expérimentales pour obtenir le produit sont identiques à celles utilisées lors de l'Exemple 1, Stade c.

### Stade d : 3-{3-(2-{[(4-Chlorophényl)sulfonyl]amino}éthyl)-5-[2-(2-{2-[4-(6-fluoro-1,2-benzisoxazol-3-yl)-1-pipéridinyl]éthoxy}phényl)éthyl] phényl}propanoate de tert-butyle

Les conditions expérimentales pour obtenir le produit sont identiques à celles utilisées lors de l'Exemple 1, Stade d.

### Stade e : Acide 3-{3-(2-{[(4-chlorophényl)sulfonyl]amino}éthyl)-5-[2-(2-{2-[4-(6-fluoro-1,2-benzisoxazol-3-yl)-1-pipéridinyl]éthoxy}phényl)éthyl]phényl} propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit est extrait à l'acétate d'éthyle. Les phases organiques sont collectées et séchées sur sulfate de magnésium. Le produit brut est purifié par chromatographie sur colonne de silice (éluant : dichlorométhane/méthanol: 95/5).

| *Microanalyse élémentaire :* | | | | | |
|---|---|---|---|---|---|
| | C | H | Cl | N | S |
| % calculé | 63,79 | 5,63 | 4,83 | 5,72 | 4,37 |
| % trouvé | 63,74 | 5,82 | 5,04 | 5,66 | 4,01 |

### EXEMPLE 10 : Chlorhydrate de l'acide 3-[3-[2-(2-{2-[4-(1,2-benzisothiazol-3-yl)-1-pipérazinyl]éthoxy}phényl)éthyl]-5-(2-{[(4-chlorophényl)sulfonyl] amino}étbyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(2-{2-[4-(1,2-Benzisothiazol-3-yl)-1-pipérazinyl]éthoxy}phényl) éthyl]-5-[2-(1,3-dioxo-1,3-dihydro-2H-isoindol-2-yl)éthyl]phényl} propanoate de tert-butyle

Le produit attendu est obtenu selon le procédé décrit dans l'Exemple 1, Stade b en remplaçant le 2-chloro-*N,N*-diméthyléthanamine par le 3-[4-(2-chloroéthyl)-4-pipérazinyl]-1,2-benzisothiazole.

### Stade c : 3-{3-(2-Aminoéthyl)-5-[2-(2-{2-[4-(1,2-benzisothiazol-3-yl)-1-pipérazinyl]éthoxy}phényl)éthyl]phényl}propanoate de tert-butyle

Les conditions expérimentales pour obtenir le produit sont identiques à celles utilisées lors de l'Exemple 1, Stade c.

### Stade d : 3-[3-[2-(2-{2-[4-(1,2-Benzisothiazol-3-yl)-1-pipérazinyl]éthoxy}phényl) éthyl]-5-(2-{[(4-chlorophényl)sulfonyl]amino}éthyl)phényl]propanoate de tert-butyle

Les conditions expérimentales pour obtenir le produit sont identiques à celles utilisées lors de l'Exemple 1, Stade d.

### Stade e : Chlorhydrate de l'acide 3-[3-[2-(2-{2-[4-(1,2-benzisothiazol-3-yl)-1-pipérazinyl]éthoxy}phényl)éthyl]-5-(2-{[(4-chlorophényl)sulfonyl] amino}éthyl)phényl]propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit attendu est filtré, lavé à l'eau puis repris dans le dichlorométhane. Après addition d'un équivalent d'une solution 1 N d'acide chlorhydrique dans l'éther éthylique, le produit précipité est filtré.

| *Microanalyse élémentaire :* | | | | | |
|---|---|---|---|---|---|
| | C | H | Cl | N | S |
| % calculé | 59,29 | 5,50 | 4,61 | 7,28 | 8,33 |
| % trouvé | 59,06 | 5,57 | 4,70 | 7,13 | 7,97 |

### EXEMPLE 11 : Acide 3-{3-(2-{[(4-chlorophényl)sulfonyl]amino}éthyl)-5-[2-(2-{2-(4-(6-fluoro-1,2-benzisothiazol-3-yl)-1-pipéridinyl]éthoxy}phényl) éthyl]phényl}propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(1,3-Dioxo-1,3-dihydro-2H-isoindol-2-yl)éthyl]-5-[2-(2-{2-[4-(6-fluoro-1,2-benzisothiazol-3-yl)-1-pipéridinyl]éthoxy}phényl)éthyl] phényl}propanoate de tert-butyle

Le produit attendu est obtenu selon le procédé décrit dans l'Exemple 1, Stade b en remplaçant le 2-chloro-*N,N*-diméthyléthanamine par le 3-[1-(2-chloroéthyl)-4-pipéridinyl]-6-fluoro-1,2-benzisothiazole.

### Stade c : 3-{3-(2-Aminoéthyl)-5-[2-(2-{2-[4-(6-fluoro-1,2-benzisothiazol-3-yl)-1-pipéridinyl]éthoxy}phényl)éthyl]phényl}propanoate de tert-butyle

Les conditions expérimentales pour obtenir le produit sont identiques à celles utilisées lors de l'Exemple 1, Stade c.

### Stade d: 3-{3-(2-{[(4-Chlorophényl)sulfonyl]amino}éthyl)-5-[2-(2-{2-[4-(6-fluoro-1,2-benzisothiazol-3-yl)-1-pipéridinyl]éthoxy}phényl)éthyl]phényl} propanoate de tert-butyle

Les conditions expérimentales pour obtenir le produit sont identiques à celles utilisées lors de l'Exemple 1, Stade d.

### Stade e : Acide 3-{3-(2-{[(4-chlorophényl)sulfonyl]amino}éthyl)-5-[2-(2-{2-[4-(6-fluoro-1,2-benzisothiazol-3-yl)-1-pipéridinyl]éthoxy}phényl)éthyl] phényl}propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit brut est purifié par précipitation, puis après filtration est lavé à l'éther. Le produit attendu est purifié par chromatographie sur colonne de silice (éluant : dichlorométhane/méthanol: 97/3).

| *Microanalyse élémentaire :* | | | | | |
|---|---|---|---|---|---|
| | C | H | Cl | N | S |
| % calculé | 62,43 | 5,51 | 4,72 | 5,60 | 8,55 |
| % trouvé | 62,19 | 5,60 | 4,86 | 5,58 | 8,08 |

### EXEMPLE 12 : Chlorhydrate de l'acide 3-[3-[2-(2-{3-[4-(1,2-benzisothiazol-3-yl)-1-pipérazinyl]propoxy}phényl)éthyl]-5-(2-{[(4-chlorophényl)sulfonyl] amino}éthyl)phényl]propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(2-{3-[4-(1,2-Benzisothiazol-3-yl)-1-pipérazinyl]propoxy} phényl)éthyl]-5-[2-(1,3-dioxo-1,3-dihydro-2H-isoindol-2-yl)éthyl] phényl}propanoate de tert-butyle

Le produit attendu est obtenu selon le procédé décrit dans l'Exemple 1, Stade b en remplaçant le 2-chloro-*N,N*-diméthyléthanamine par le 3-[4-(3-chloropropyl)-1-pipérazinyl]-1,2-benzisothiazole.

### Stade c : 3-{3-(2-Aminoéthyl)-5-[2-(2-{3-[4-(1,2-benzisothiazol-3-yl)-1-pipérazinyl]propoxy}phényl)éthyl]phényl}propanoate de tert-butyle

Les conditions expérimentales pour obtenir le produit sont identiques à celles utilisées lors de l'Exemple 1, Stade c.

### Stade d : 3-[3-[2-(2-{3-[4-(1,2-Benzisothiazol-3-yl)-1-pipérazinyl]propoxy} phényl)éthyl]-5-(2-{[(4-chlorophényl)sulfonyl]amino}éthyl)phényl] propanoate de tert-butyle

Les conditions expérimentales pour obtenir le produit sont identiques à celles utilisées lors de l'Exemple 1, Stade d.

### Stade e : Chlorhydrate de l'acide 3-[3-[2-(2-{3-[4-(1,2-benzisothiazol-3-yl)-1-pipérazinyl]propoxy}phényl)éthyl]-5-(2-{[(4-chlorophényl)sulfonyl] amino}éthyl)phényl]propanoïque

0,004 moles du composé obtenu au stade précédent sont mises en solution dans 25 ml d'acide trifluoroacétique. Le milieu réactionnel est agité pendant 30 minutes puis l'acide trifluoroacétique est évaporé. Le produit brut est repris dans une solution de soude 2 N. Après extraction par de l'éther éthylique, la phase aqueuse est acidifiée par de l'acide acétique jusqu'à pH 4-5. Le produit brut est purifié par précipitation, puis après filtration est lavé à l'éther. Le produit attendu est purifié par chromatographie sur colonne de silice (éluant : dichlorométhane/méthanol/ammoniaque : 95/5/0,5). Le produit attendu est repris dans le dichlorométhane. Après addition d'un équivalent d'une solution 1 N d'acide chlorhydrique dans l'éther, le produit précipite et est filtré.

| *Microanalyse élémentaire :* | | | | | |
|---|---|---|---|---|---|
| | C | H | Cl | N | S |
| % calculé | 59,76 | 5,66 | 9,05 | 7,15 | 8,18 |
| % trouvé | 59,76 | 5,67 | 9,05 | 6,97 | 7,54 |

### EXEMPLE 13 : Acide 3-{3-(2-{[(4-chlorophényl)sulfonyl]amino}éthyl)-5-[2-(2-{3-[4-(6-fluoro-1,2-benzisoxazol-3-yl)-1-pipéridinyl]propoxy}phényl) éthyl]phényl}propanoïque

### Stade a : 3-{3-[2-(1,3-Dioxo-1,3-dihydro-2H-isoindol-2-yl)éthyl]-5-[2-(2-hydroxyphényl)éthyl]phényl}propanoate de tert-butyle

Le procédé expérimental est identique à celui de l'Exemple 1, Stade a.

### Stade b : 3-{3-[2-(1,3-Dioxo-1,3-dihydro-2H-isoindol-2-yl)éthyl]-5-[2-(2-{3-[4-(6-fluoro-1,2-benzisoxazol-3-yl)-1-pipéridinyl]propoxy}phényl)éthyl] phényl}propanoate de tert-butyle

Le produit attendu est obtenu selon le procédé décrit dans l'Exemple 1, Stade b en remplaçant le 2-chloro-*N,N*-diméthyléthanamine par le 3-[1-(3-chloropropyl)-4-pipéridinyl]-6-fluoro-1,2-benzisoxazole.

### Stade c : 3-{3-(2-Aminoéthyl)-5-[2-(2-{3-[4-(6-fluoro-1,2-benzisoxazol-3-yl)-1-pipéridinyl]propoxy}phényl)éthyl]phényl}propanoate de tert-butyle

Les conditions expérimentales pour obtenir le produit sont identiques à celles utilisées lors de l'Exemple 1, Stade c.

### Stade d : 3-{3-(2-{[(4-Chlorophényl)sulfonyl]amino}éthyl)-5-[2-(2-{3-[4-(6-fluoro-1,2-benzisoxazol-3-yl)-1-pipéridinyl]propoxy}phényl)éthyl] phényl}propanoate de tert-butyle

Les conditions expérimentales pour obtenir le produit sont identiques à celles utilisées lors de l'Exemple 1, Stade d.

### Stade e : Acide 3-{3-(2-{[(4-chlorophényl)sulfonyl]amino}éthyl)-5-[2-(2-{3-[4-(6-fluoro-1,2-benzisoxazol-3-yl)-1-pipéridinyl]propoxy}phényl)éthyl] phényl}propanoïque

Le produit attendu est obtenu selon la même méthode que celle décrite dans l'Exemple 8, Stade e, à l'éluant près lors de la purification par chromatographie sur colonne de silice (dichlorométhane/méthanol/ammoniaque : 95/5/0,5).

| *Microanalyse élémentaire :* | | | | | |
|---|---|---|---|---|---|
| | C | H | Cl | N | S |
| % calculé | 64,20 | 5,79 | 4,74 | 5,62 | 4,28 |
| % trouvé | 63,89 | 5,95 | 4,41 | 5,46 | 4,12 |

### EXEMPLE 14 : Acide 3-[3-(2-{[(4-chlorophényl)sulfonyl]amino}éthyl)-5-({4-[2-(diméthylamino)éthoxy]phénoxy}méthyl)phényl]propanoïque

### Stade a : 3-[3-[2-(1,3-Dioxo-1,3-dihydro-2H-isoindol-2-yl)éthyl]-5-(hydroxyméthyl)phényl]propanoate de tert-butyle

L'hydrogénation catalytique du produit de la Préparation B, Stade e, est réalisé dans les mêmes conditions expérimentales que dans l'Exemple 1, Stade a.

### Stade b : 3-{3-(Bromométhyl)-5-[2-(1,3-dioxo-1,3-dihydro-2H-isoindol-2-yl) éthyl]phényl}propanoate de tert-butyle

A 0,052 moles du composé obtenu au stade précédent en solution dans 320 ml de dichlorométhane sont ajoutées 0,063 moles de triphénylphosphine puis, lentement, une solution de 0,063 moles de tétrabromure de carbone en solution dans 85 ml de dichlorométhane en maintenant la température inférieure à 30°C. Le milieu est agité 15 heures puis mis à sec. Le produit brut est purifié par chromatographie (éluant : cyclohexane/acétate d'éthyle : 80/20).

### Stade c : 3-{3-({4-[2-(Diméthylamino)éthoxy]phénoxy}méthyl)-5-[2-(1,3-dioxo-1,3-dihydro-2H-isoindol-2-yl)éthyl]phényl}propanoate de tert-butyle

A 0,0196 moles d'hydrure de sodium, préalablement lavé au pentane, dans 90 ml de toluène sont ajoutées 0,0083 moles de 4-[2-(diméthylamino)éthoxy]phénol en suspension dans 15 ml de toluène. Le milieu est porté au reflux pendant 1 heure, puis 0,0064 moles du composé obtenu au stade précédent en solution dans 90 ml de toluène sont introduits. Le reflux est maintenu pendant 4 heures. Après addition d'eau et extraction par de l'éther éthylique, les phases organiques sont lavées par une solution de soude 1 N et séchées sur sulfate de magnésium. Après filtration, le produit brut est utilisé sans autre purification.

### Stade d : Acide 3-[3-(2-{[(4-chlorophényl)sulfonyl]amino}éthyl)-5-({4-[2-(diméthylamino)éthoxy]phénoxy}méthyl)phényl]propanoïque

A partir du produit précédemment synthétisé, les méthodes expérimentales de déprotection de l'amine, de l'addition du chlorure de benzène sulfonyle et enfin de l'hydrolyse de l'ester sont identiques à celles utilisées respectivement dans l'Exemple 1, Stade c, Stade d et enfin Stade e.

| *Microanalyse élémentaire :* | | | | | |
|---|---|---|---|---|---|
| | C | H | Cl | N | S |
| % calculé | 59,94 | 5,93 | 6,32 | 4,99 | 5,71 |
| % trouvé | 59,95 | 6,04 | 6,94 | 4,97 | 5,43 |

### EXEMPLE 15 : Acide 3-[3-(2-{[(4-chlorophényl)sulfonyl]amino}éthyl)-5-({2-[2-(diméthylamino)éthoxy]phénoxy}méthyl)phényl]propanoïque

### Stade a : 3-[3-[2-(1,3-Dioxo-1,3-dihydro-2H-isoindol-2-yl)éthyl]-5-(hydroxyméthyl)phényl]propanoate de tert-butyle

Le procédé expérimental est identique à celui de l'Exemple 14, Stade a.

### Stade b : 3-{3-(Bromométhyl)-5-[2-(1,3-dioxo-1,3-dihydro-2H-isoindol-2-yl)éthyl] phényl}propanoate de tert-butyle

Le procédé expérimental est identique à celui de l'Exemple 14, Stade b.

### Stade c : 3-{3-({2-[2-(Diméthylamino)éthoxy]phénoxy}méthyl)-5-[2-(1,3-dioxo-1,3-dihydro-2H-isoindol-2-yl)éthyl]phényl}propanoate de tert-butyle

Le produit attendu est obtenu selon le procédé décrit dans l'Exemple 14, Stade c, en remplaçant le 4-[2-(diméthylamino)éthoxy]phénol par le 2-[2-(diméthylamino) éthoxy]phénol.

### Stade d : Acide 3-[3-(2-{[(4-chlorophényl)sulfonyl]amino}éthyl)-5-({2-[2-(diméthylamino)éthoxy]phénoxy}méthyl)phényl]propanoïque

Le procédé expérimental est identique à celui de l'Exemple 14, Stade d.

| *Microanalyse élémentaire :* | | | | | |
|---|---|---|---|---|---|
| | C | H | Cl | N | S |
| % calculé | 59,94 | 5,93 | 6,32 | 4,99 | 5,71 |
| % trouvé | 60,34 | 6,16 | 6,77 | 4,91 | 5,31 |

### EXEMPLE 16 : Acide 3-[3-(2-{[(4-chlorophényl)sulfonyl]amino}éthyl)-5-({2-[3-(diméthylamino)propoxy]phénoxy}méthyl)phényl]propanoïque

### Stade a : 3-[3-[2-(1,3-Dioxo-1,3-dihydro-2H-isoindol-2-yl)éthyl]-5-(hydroxyméthyl)phényl]propanoate de tert-butyle

Le procédé expérimental est identique à celui de l'Exemple 14, Stade a.

### Stade b : 3-{3-(Bromométhyl)-5-[2-(1,3-dioxo-1,3-dihydro-2H-isoindol-2-yl) éthyl]phényl}propanoate de tert-butyle

Le procédé expérimental est identique à celui de l'Exemple 14, Stade b.

### Stade c: 3-{3-({2-[3-(Diméthylamino)propoxy]phénoxy}méthyl)-5-[2-(1,3-dioxo-1,3-dihydro-2H-isoindol-2-yl)éthyl]phényl}propanoate de tert-butyle

Le produit attendu est obtenu selon le procédé décrit dans l'Exemple 14, Stade c, en remplaçant le 4-[2-(diméthylamino)éthoxy]phénol par le 2-[3-(diméthylamino) propoxy]phénol.

### Stade d : Acide 3-[3-(2-{[(4-chlorophényl)sulfonyl]amino}éthyl)-5-({2-[3-(diméthylamino)propoxy]phénoxy}méthyl)phényl]propanoïque

Le procédé expérimental est identique à celui de l'Exemple 14, Stade d.

| *Microanalyse élémentaire :* | | | | | |
|---|---|---|---|---|---|
| | C | H | Cl | N | S |
| % calculé | 60,56 | 6,13 | 6,16 | 4,87 | 5,58 |
| % trouvé | 61,57 | 6,59 | 6,09 | 4,62 | 5,52 |

### EXEMPLE 17 : Chlorhydrate d'acide 3-(3-(2-{[(4-chlorophényl)sulfonyl]amino} éthyl)-5-{2-[3-(diméthylamino)propoxy]benzyl}phényl)propanoïque

### Stade a : 3-{3-(Bromométhyl)-5-[2-(1,3-dioxo-1,3-dihydro-2H-isoindol-2-yl)éthyl] phényl}propanoate de tert-butyle

Le procédé expérimental est identique à celui de l'Exemple 14, Stade b.

### Stade b : 3-{3-[2-(Benzyloxy)benzyl]-5-[2-(1,3-dioxo-1,3-dihydro-2H-isoindol-2-yl)éthyl]phényl}propanoate de tert-butyle

A 0,017 moles du composé obtenu au stade précédent en solution dans 130 ml d'un mélange toluène/éthanol : 80/50 sont additionnés successivement 0,017 moles d'acide 2-benzyloxyphényl boronique, 34 ml d'une solution 2 M de Na₂CO₃ et 0,85 moles de tétrakis-triphénylphosphine palladium. Le milieu réactionnel est porté au reflux pendant 3 heures 30 puis refroidi. Après addition d'eau et de toluène, le milieu est décanté et extrait 3 fois au toluène. Les phases organiques sont lavées 2 fois par une solution saturée de NaCl puis séchées sur sulfate de magnésium. Après évaporation des solvants, le produit brut est utilisé sans autre purification.

### Stade c: 3-[3-[2-(1,3-Dioxo-1,3-dihydro-2H-isoindol-2-yl)éthyl]-5-(2-hydroxybenzyl)phényl]propanoate de tert-butyle

A 0,0125 moles du composé obtenu au stade précédent dans 90 ml d'un mélange éthanol/THF : 50/40 sont additionnés 1,4 grammes de dihydroxyde de palladium. Le milieu est mis en présence d'hydrogène à température ambiante pendant 2 heures. Le milieu est filtré et le solvant évaporé. Le produit est utilisé sans autre purification.

### Stade d : 3-{3-{2-[3-(Diméthylamino)propoxy]benzyl}-5-[2-(1,3-dioxo-1,3-dihydro-2H-isoindol-2-yl)éthyl]phényl}propanoate de tert-butyle

Le procédé expérimental est identique à celui de l'Exemple 2, Stade b.

### Stade e : Chlorhydrate d'acide 3-(3-(2-{[(4-chlorophényl)sulfonyl]amino}éthyl)-5-{2-[3-(diméthylamino)propoxy]benzyl}phényl)propanoïque

La déprotection de l'amine, l'addition du chlorure de benzène sulfonyle et enfin l'hydrolyse de l'ester sont identiques aux méthodes expérimentales utilisées respectivement dans l'Exemple 1, Stade c, Stade d et enfin Stade e.

| *Microanalyse élémentaire :* | | | | | |
|---|---|---|---|---|---|
| | C | H | Cl | N | S |
| % calculé | 60,32 | 6,21 | 9,21 | 4,85 | 5,55 |
| % trouvé | 59,31 | 6,34 | 8,88 | 4,57 | 5,61 |

### EXEMPLE 18 : Acide 3-[3-(2-{[(4-chlorophényl)sulfonyl]amino}éthyl)5-(3-{2-[3-(diméthylamino)propoxy]phényl}propyl)phényl]propanoïque

### Stade a : 3-{3-{3-[2-(Benzyloxy)phényl]propyl}-5-[2-(1,3-dioxo-1,3-dihydro-2H-isoindol-2-yl)éthyl]phényl}propanoate de tert-butyle

Le procédé expérimental est identique à celui de l'Exemple 1, Stade a en partant à la place du composé de la Préparation A, Stade e, du composé de la Préparation C, Stade e .

### Stade b : 3-{3-[2-(1,3-Dioxo-1,3-dihydro-2H-isoindol-2-yl)éthyl]-5-[3-(2-hydroxyphényl)propyl]phényl}propanoate de tert-butyle

A 8,40 mmoles du composé obtenu au stade précédent dissout dans 80 ml d'éthanol, sont ajoutés 10% d'hydroxyde de palladium. Le milieu est hydrogéné pendant 2 heures et demi et le solvant est évaporé.

### Stade c: 3-{3-(3-{2-[3-(Diméthylamino)propoxy]phényl}propyl)-5-[2-(1,3-dioxo-1,3-dihydro-2H-isoindol-2-yl)éthyl]phényl}propanoate de tert-butyle

Le procédé expérimental est identique à celui de l'Exemple 2, Stade b à partir du composé préparé lors du stade précédent.

### Stade d : 3-[3-(2-Aminoéthyl)-5-(3-{2-[3-(diméthylamino)propoxy]phényl}propyl)-phényl]propanoate de tert-butyle

Le procédé expérimental est identique à celui de l'Exemple 1, Stade c à partir du composé préparé lors du stade précédent.

### Stade e : Acide 3-[3-(2-{[(4-chlorophényl)sulfonyl]amino}éthyl)-5-(3-{2-[3-(diméthylamino)propoxy]phényl}propyl)phényl]propanoïque

La déprotection de l'amine, l'addition du chlorure de benzène sulfonyle et enfin l'hydrolyse de l'ester sont identiques aux méthodes expérimentales utilisées respectivement dans l'Exemple 1, Stade c, Stade d et enfin Stade e.

| *Microanalyse élémentaire :* | | | | | |
|---|---|---|---|---|---|
| | C | H | Cl | N | S |
| % calculé | 63,41 | 6,69 | 6,04 | 4,77 | 5,46 |
| % trouvé | 62,93 | 6,69 | 6,60 | 4,70 | 5,35 |

### ETUDE PHARMACOLOGIQUE

### EXEMPLE A : Agrégation plaquettaire chez l'homme

Le sang veineux est obtenu de volontaires humains n'ayant pas pris de l'aspirine pendant au moins 14 jours précédent l'expérience. Le sang est prélevé sur citrate de sodium (0.109 M) (1 vol. de citrate sur 9 vol. de sang). Le plasma riche en plaquettes (PRP) est obtenu après centrifugation (20°C) à 200 g pendant 10 minutes. Le nombre de plaquettes est en moyenne de 250000 PL/mm³. Le PRP est conservé à la température de la pièce jusqu'au moment du test et est utilisé dans les 2 heures qui suivent le prélèvement. L'agoniste TXA₂, le U46619 est utilisé à la concentration de 1 µM et la 5-hydroxytryptamine à la concentration de 10 µM, ce dernier en présence d'adénosine diphosphate à 0.3 µM et d'adrénaline à 1 µM.

Les composés de l'invention inhibent l'agrégation plaquettaire induite par l'agoniste TXA₂ ainsi que celle produite par la 5-hydroxytryptamine. A titre d'exemple les IC₅₀ du composé de l'exemple 2 sur les deux expériences sont respectivement de 3,3 µM et de 0,96 µM.

Les valeurs indiquent que les composés de l'invention sont de puissants anti-agrégants plaquettaires agissant de façon balancée sur les deux voies d'activation, celle du TXA₂ et celle de la sérotonine.

### EXEMPLE B : Composition pharmaceutique

| Formule de préparation pour 1000 comprimés dosés à 5 mg : | |
|---|---|
| Composé de l'exemple 4 | 5 g |
| Hydroxypropylméthylcellulose | 2 g |
| Amidon de blé | 10 g |
| Lactose | 100 g |
| Stéarate de magnésium | 3 g |

## Revendications

1. Composés de formule (I) : 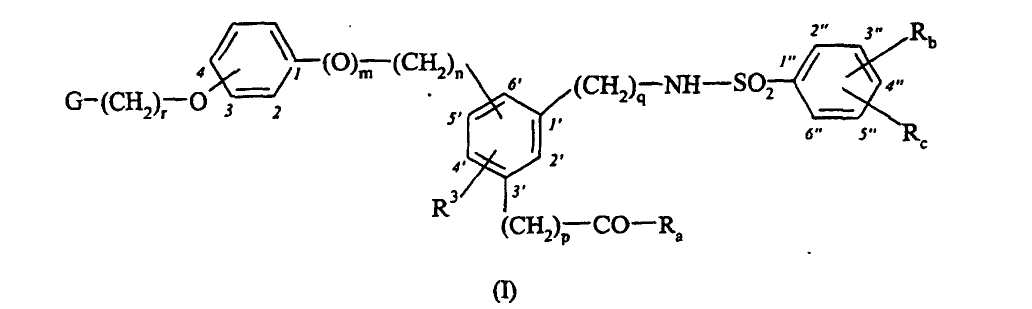 dans laquelle :
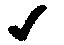 G représente un groupement tel que : 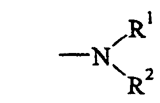 où R¹ et R² représentent indépendamment un atome d'hydrogène, un groupement alkyle, cycloalkyle, aryle éventuellement substitué, arylalkyle éventuellement substitué, cycloalkyle, cycloalkylalkyle, hétéroaryle éventuellement substitué, ou hétéroarylalkyle éventuellement substitué,
ou bien,
R¹ et R² forment ensemble avec l'atome d'azote un groupement hétérocycloalkyle de formule 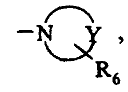 de 5 à 7 chaînons dans lequel Y représente un atome d'azote, d'oxygène ou un groupement CH₂ et R₆ représente un atome d'hydrogène, un groupement alkyle, cycloalkyle, cycloalkylalkyle, aryle éventuellement substitué, arylalkyle éventuellement substitué, arylcarbonyle éventuellement substitué, arylcarbonylalkyle éventuellement substitué, diarylalkyle éventuellement substitué, diarylalkényle éventuellement substitué, hétéroaryle éventuellement substitué, hétéroarylalkyle éventuellement substitué, hétéroarylcarbonyle éventuellement substitué, ou hétéroarylcarbonylalkyle éventuellement substitué,
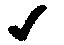 R³ représente un atome d'hydrogène, groupement alkyle, ou phényle éventuellement substitué,
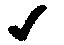 Rₐ représente un groupement hydroxy, alkoxy, aryloxy éventuellement substitué, arylalkyloxy éventuellement substitué, amino, alkylamino, dialkylamino, arylamino éventuellement substitué, arylalkylamino éventuellement substitué,
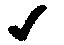 R_{b} et R_{c}, identiques ou différents, représentent un atome d'hydrogène, un atome d'halogène, un groupement alkyle, un groupement alkoxy, un groupement hydroxy ou un groupement trihalogénoalkyle,
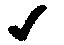 m est un entier compris inclusivement entre 0 et 1,
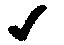 n et q sont des entiers identiques ou différents compris inclusivement entre 0 et 6,
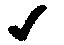 p et r sont des entiers identiques ou différents compris inclusivement entre 1 et 6,
leurs énantiomères, diastéréoisomères ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkoxy désigne un groupement alkyle-oxy, contenant de 1 à 6 atomes de carbone, linéaire ou ramifié,
- le terme trihalogénoalkyle désigne un chaîne carbonée, contenant de 1 à 3 atomes de carbone et de 1 à 3, identiques ou différents, atomes d'halogène,
- le terme alkényle désigne une chaîne de 2 à 6 atomes de carbone contenant de 1 à 3 doubles liaisons,
- le terme cycloalkyle désigne un groupement cyclique saturé contenant de 3 à 8 atomes de carbone,
- le terme aryle désigne un groupement phényle ou naphtyle,
- le terme hétéroaryle désigne un groupement monocyclique aromatique, ou bicyclique dans lequel au moins un des cycles est aromatique, contenant 5 à 11 chaînons, et 1 à 5 hétéroatomes choisis parmi azote, oxygène et soufre,
- les termes diarylalkyle et diarylalkényle désignent respectivement des groupements alkyle et alkényle tels que définis précédemment, substitués par deux groupements aryles, identiques ou différents, tels que définis précédemment,
- le terme "substitué" associé aux expressions phényle, aryle, arylalkyle, arylcarbonyle, arylcarbonylalkyle, diarylalkyle, diarylalkényle, hétéroaryle, hétéroarylalkyle, hétéroarylcarbonyle, hétéroarylcarbonylalkyle, arylamino et arylalkylamino signifie que les groupements concernés sont substitués sur la partie aromatique par un ou deux substituants, identiques ou différents, choisis parmi les atomes d'halogène et les groupements alkyle, alkoxy, hydroxy, cyano, nitro, amino (éventuellement substitué par un ou deux groupements alkyle) et -C(O)R_{d} avec R_{d} représentant un groupement choisi parmi hydroxy, alkoxy et amino, étant entendu que les groupements hétéroaryle et hétéroarylalkyle peuvent être en plus substitués par un groupement oxo sur la partie non-aromatique de l'hétéroaryle.

2. Composés de formule (I) selon la revendication 1 pour lesquels p vaut 2, leurs énantiomères, diastéréoisomères ainsi que leurs sels d'addition à un acide ou à une base pharmaceutiquement acceptable.

3. Composés de formule (I) selon une quelconque des revendications 1 ou 2 pour lesquels q vaut 2, leurs énantiomères, diastéréoisomères ainsi que leurs sels d'addition à un acide ou à une base pharmaceutiquement acceptable.

4. Composés de formule (I) selon une quelconque des revendications 1 à 3 pour lesquels R³ représente un atome d'hydrogène, leurs énantiomères, diastéréoisomères ainsi que leurs sels d'addition à un acide ou à une base pharmaceutiquement acceptable.

5. Composés de formule (I) selon une quelconque des revendications 1 à 4 pour lesquels Rₐ représente un groupement hydroxy, leurs énantiomères, diastéréoisomères ainsi que leurs sels d'addition à un acide ou à une base pharmaceutiquement acceptable.

6. Composés de formule (I) selon une quelconque des revendications 1 à 5 pour lesquels R_{b} représente un atome d'halogène, leurs énantiomères, diastéréoisomères ainsi que leurs sels d'addition à un acide ou à une base pharmaceutiquement acceptable.

7. Composés de formule (I) selon une quelconque des revendications 1 à 6 pour lesquels R_{c} représente un atome d'hydrogène, leurs énantiomères, diastéréoisomères ainsi que leurs sels d'addition à un acide ou à une base pharmaceutiquement acceptable.

8. Composés de formule (I) selon une quelconque des revendications 1 à 7 pour lesquels p et q valent chacun 2, Rₐ représente un groupement hydroxy, R³ et R_{c} représentent chacun un atome d'hydrogène, R_{b} représente un atome d'halogène, et G représente un groupement amino, dialkylamino, arylalkylamino, ou un groupement hétérocycloalkyle de formule : 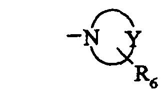 dans laquelle Y représente un atome d'azote, d'oxygène, ou un groupement CH₂ et R₆ est choisi parmi un atome d'hydrogène, les groupements aryle éventuellement substitué et hétéroaryle éventuellement substitué, leurs énantiomères, diastéréoisomères ainsi que leurs sels d'addition à un acide ou à une base pharmaceutiquement acceptable.

9. Composés de formule (I) selon une quelconque des revendications 1 à 8 pour lesquels G représente un groupement dialkylamino, leurs énantiomères, diastéréoisomères ainsi que leurs sels d'addition à un acide ou à une base pharmaceutiquement acceptable.

10. Composés de formule (I) selon une quelconque des revendications 1 à 8 pour lesquels G représente un groupement hétérocycloalkyle substitué 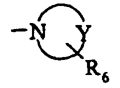 dans lequel :
• 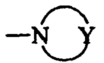 représente un groupement à 5 ou 6 chaînons tels que : pyrolyle, morpholino, pipéridyle ou pipérazinyle,
• et R₆ substitué sur un atome de carbone ou d'azote de l'hétérocycloalkyle, représente un atome d'hydrogène, un substituant phényle éventuellement substitué par un atome d'halogène, ou un groupement hétéroaryle à 9 chaînons comportant un à deux hétéroatomes choisis parmi l'atome d'azote, d'oxygène et de soufre, et éventuellement substitué par un atome d'halogène,
leurs énantiomères, diastéréoisomères ainsi que leurs sels d'addition à un acide ou à une base pharmaceutiquement acceptable.

11. Composés de formule (I) selon une quelconque des revendications 1 à 8 ou 10 pour lesquels le groupement hétéroaryle éventuellement substitué représente un groupement benzisoxazolyle éventuellement substitué par un atome d'halogène ou benzisothiazolyle éventuellement substitué par un atome d'halogène, leurs énantiomères, diastéréoisomères ainsi que leurs sels d'addition à un acide ou à une base pharmaceutiquement acceptable.

12. Composés de formule (I) selon une quelconque des revendications 1 à 11 pour lesquels, le substituant G-(CH₂)ᵣ-O- est relié à un des deux atomes de carbone *2* ou *4* du groupement phényle, leurs énantiomères, diastéréoisomères ainsi que leurs sels d'addition à un acide ou à une base pharmaceutiquement acceptable.

13. Composés de formule (I) selon une quelconque des revendications 1 à 12 pour lesquels, le substituant 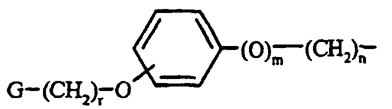 est relié à l'atome de carbone 5' du groupement phényle, leurs énantiomères, diastéréoisomères ainsi que leurs sels d'addition à un acide ou à une base pharmaceutiquement acceptable.

14. Composés de formule (I) selon une quelconque des revendications 1 à 13 pour lesquels, R_{b} est relié à l'atome de carbone 4" du groupement phényle, leurs énantiomères, diastéréoisomères ainsi que leurs sels d'addition à un acide ou à une base pharmaceutiquement acceptable.

15. Composé de formule (I) selon les revendications à 9 et 12 à 14 qui est l'acide 3-[3-(2-{[(4-chlorophényl)sulfonyl]amino}éthyl)-5-(2-{2-[3-(diméthylamino)propoxy]-phényl}éthyl)phényl] propanoïque.

16. Composés de formule (I) selon les revendications 1 à 8, 10, 12 à 14 qui sont :
- l'acide 3-[3-(2-{[(4-chlorophényl)sulfonyl]amino}éthyl)-5-(2-{2-[2-(1-pyrrolidinyl)éthoxy]phényl} éthyl)phényl]propanoïque,
- et l'acide 3-[3-(2-{[(4-chlorophényl)sulfonyl]amino}éthyl)-5-(2-{2-[2-(4-morpholinyl)éthoxy]phényl}éthyl) phényl]propanoïque.

17. Procédé de préparation des composés de formule (I) selon la revendication 1 **caractérisé en ce que** :
① lorsque les composés de formule (I) que l'on souhaite obtenir sont tels que m=0 et n différent de 1, l'on utilise comme produit de départ un composé de formule (II) : 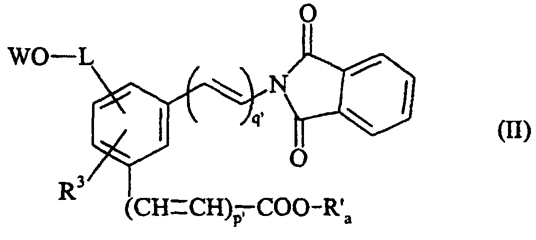 dans laquelle R³ a la même signification que dans la formule (I), R'ₐ représente un groupement alkyle (C₁-C₆) linéaire ou ramifié, WO-L représente un groupement de formule: 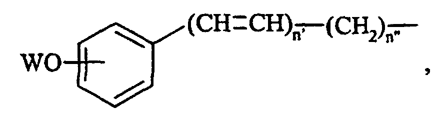 avec W représentant un atome d'hydrogène ou un groupement benzyle, p' et q' sont des entiers compris entre 0 et 3, n'+n"= un entier compris entre 0 et 3 qui lorsque n'vaut 0 alors n''vaut 0, 2 ou 3,
- qui est réduit catalytiquement pour conduire après débenzylation, lorsque W représente un groupement benzyle, au composé de formule (III) : 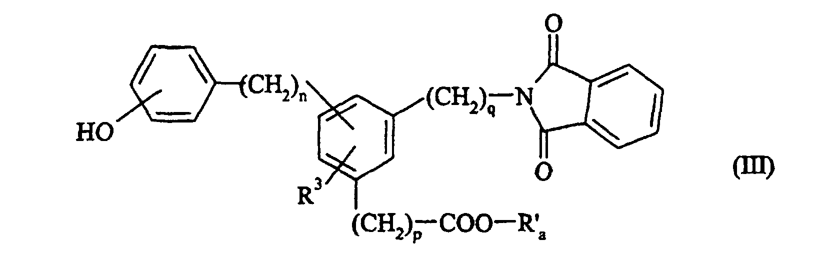 dans laquelle R³, n, p et q ont la même signification que dans la formule (I), R'ₐ représente un groupement alkyle (C₁-C₆) linéaire ou ramifié,
- qui se condense en milieu basique avec un halogénure de formule : G-(CH₂)ᵣ-X, dans laquelle X représente un atome d'halogène pour conduire au composé de formule (IV) : 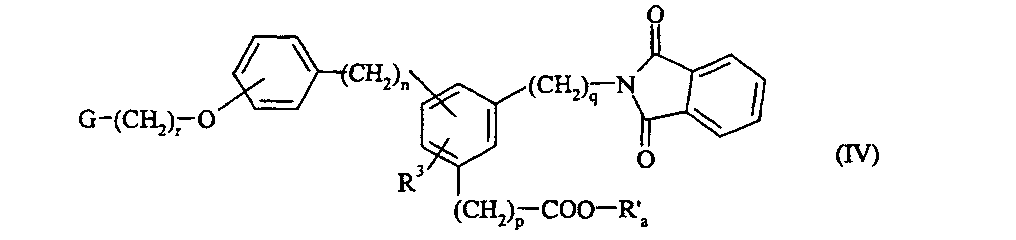 dans laquelle G, R³, n, p, q et r ont la même signification que dans la formule (I), R'ₐ représente un groupement alkyle (C₁-C₆) linéaire ou ramifié,
- qui est déprotégé par clivage du groupement phtalimido en présence d'hydrazine pour conduire à l'amine correspondante qui, à son tour, réagit avec un halogénure de benzène sulfonyle éventuellement substitué de formule : 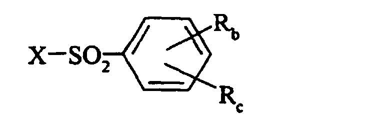
dans laquelle R_{b} et R_{c} ont la même signification que dans la formule (I) et X représente un atome d'halogène,
pour donner le composé de formule (I/a), cas particulier des composés (I) : 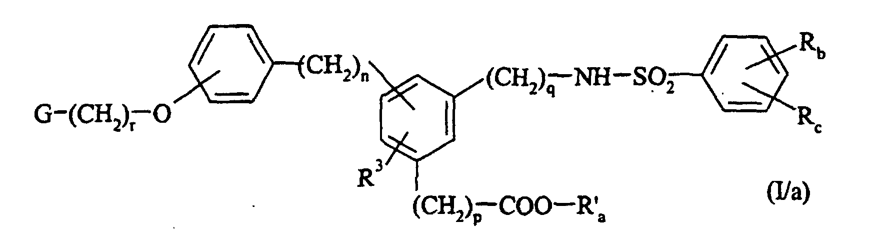 dans laquelle G, R_{b}, R_{c}, R³, r, n, p et q ont la même signification que dans la formule (I), R'ₐ représente un groupement alkyle (C₁-C₆) linéaire ou ramifié,
② lorsque les composés de formule (I) que l'on souhaite obtenir sont tels que m=1, ou m=0 avec n=1, l'on utilise comme produit de départ un composé de formule (II/a) : 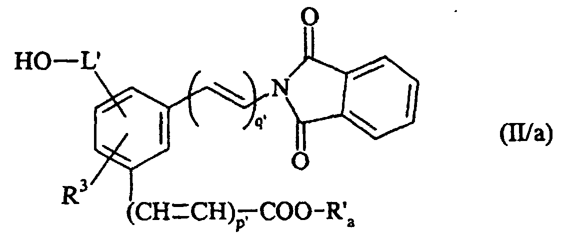 dans laquelle R³ a la même signification que dans la formule (I), R'ₐ représente un groupement alkyle (C₁-C₆) linéaire ou ramifié, HO-L' représente un groupement de formule : HO-(CH₂)ₙ-, q', p' sont des nombres entiers compris entre 0 et 3, et n est un nombre entier compris entre 0 et 6,
- qui est réduit catalytiquement et halogéné pour conduire au composé (V) : 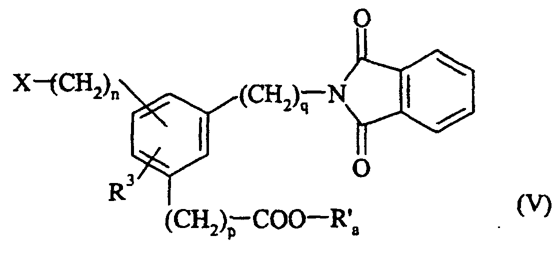
dans laquelle R³, n, p et q ont la même signification que dans la formule (I), R'ₐ représente un groupement alkyle (C₁-C₆) linéaire ou ramifié, X représente un atome d'halogène,
composé de formule (V) qui subit :
- soit, lorsque m=1, l'attaque nucléophile d'un composé de formule : 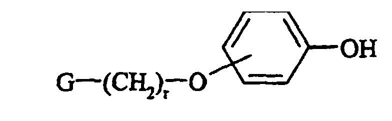 pour conduire au composé de formule (VI): 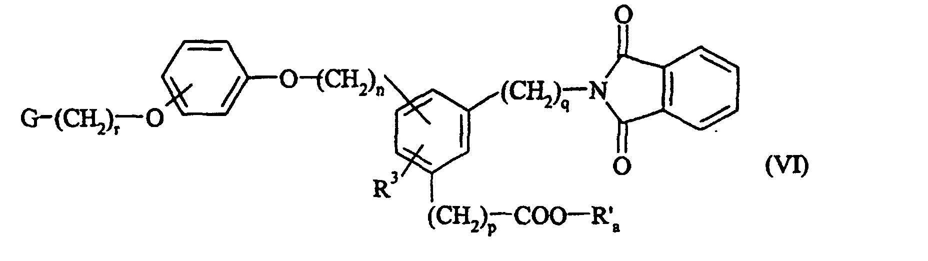 dans laquelle G, R³, n, p, q et r ont la même signification que dans la formule (I), R'ₐ représente un groupement alkyle (C₁-C₆) linéaire ou ramifié,
- qui est déprotégé par clivage du groupement phtalimido en présence d'hydrazine pour conduire à l'amine correspondante qui, à son tour, réagit avec un halogénure de benzène sulfonyle éventuellement substitué de formule : 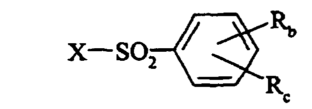 dans laquelle X représente un atome d'halogène, pour donner le composé de formule (I/b), cas particulier des composés de formule (I) : 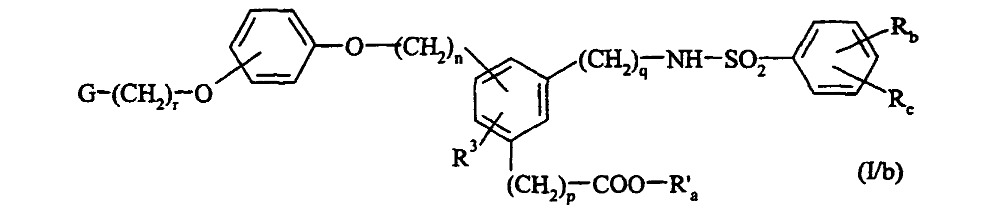 dans laquelle G, R_{b}, R_{c}, R³, n, p, q et r ont la même signification que dans la formule (I), R'ₐ représente un groupement alkyle (C₁-C₆) linéaire ou ramifié,
- **soit,** lorsque m=0 et n=1, l'action d'un composé de formule 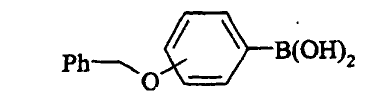 pour conduire après débenzylation au composé de formule (VII) : 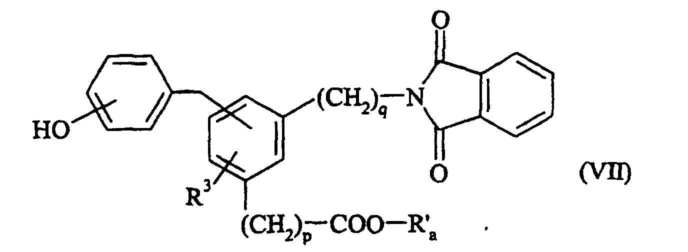 dans laquelle R³, p et q ont la même signification que dans la formule (I), R'ₐ représente un groupement alkyle (C₁-C₆) linéaire ou ramifié,
- qui se condense en milieu basique avec un halogénure de formule G-(CH₂)ᵣ-X, dans laquelle X représente un atome d'halogène pour conduire au composé de formule (VIII) : 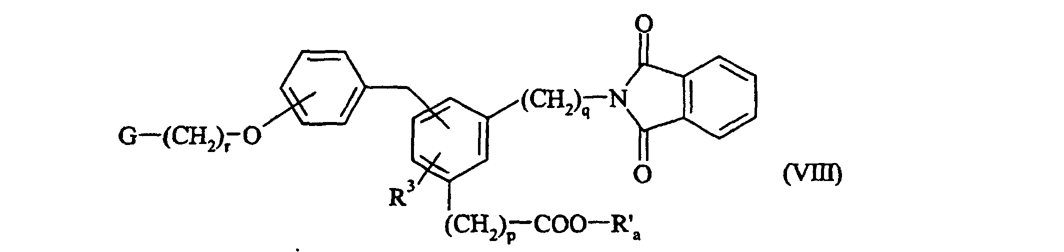 dans laquelle G, R³, p, q et r ont la même signification que dans la formule (I), R'ₐ représente un groupement alkyle (C₁-C₆) linéaire ou ramifié,
- qui est déprotégé par clivage du groupement phtalimido en présence d'hydrazine pour conduire à l'amine correspondante qui, à son tour, réagit avec un halogénure de benzène sulfonyle éventuellement substitué de formule : 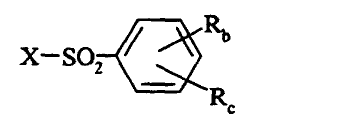 pour donner le composé de formule (I/c), cas particulier des composés (I) : 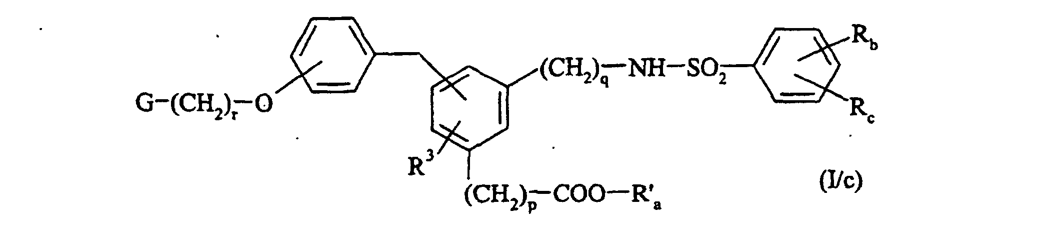 dans laquelle G, R_{b}, R_{c}, R³, p, q et r ont la même signification que dans la formule (I), R'ₐ représente un groupement alkyle (C₁-C₆) linéaire ou ramifié,
composés de formule (I/a), (I/b) ou (I/c), qui peuvent être soumis à une hydrolyse de la fonction ester, en milieu basique ou acide selon les groupements réactifs présents sur la molécule, pour conduire au composé de formule (I/d) : 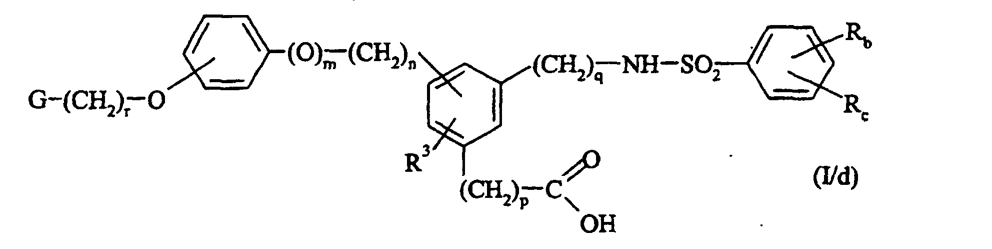 cas particulier des composés de formule (I) G, R_{b}, R_{c}, R³, m, n, p, q et r sont tels que définis dans la formule (I),
composés (I/a), (I/b), (I/c) et (I/d) formant la totalité des composés de formule (I), et :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable,
étant entendu qu'à tout moment jugé opportun au cours du procédé précédemment décrit, la fonction acide carboxylique peut être estérifiée ou bien la fonction ester carboxylique peut être hydrolysée en acide correspondant, ce dernier pouvant être à nouveau transformé en un autre ester pour les besoins de la synthèse.

18. Compositions pharmaceutiques contenant comme principe actif au moins un composé selon l'une quelconque des revendications 1 à 16 seul ou en combinaison avec un ou plusieurs excipients ou véhicules inertes, non toxiques, pharmaceutiquement acceptables.

19. Compositions pharmaceutiques selon la revendication 18 contenant au moins un principe actif selon l'une quelconque des revendications 1 à 16 utiles pour la fabrication de médicaments utiles comme antagonistes des récepteurs du TXA₂ et des récepteurs 5-HT₂.

20. Compositions pharmaceutiques selon la revendication 18 contenant au moins un principe actif selon l'une quelconque des revendications 1 à 16 utiles pour la fabrication de médicaments utiles dans le traitement des maladies cardiovasculaires athéro-thrombotiques telles que l'infarctus du myocarde, l'angine de poitrine, les accidents vasculaires cérébraux, la maladie de Raynaud, ou encore de l'asthme, des brochospasmes, de la migraine et des maladies veineuses.

## Patentansprüche

1. Verbindungen der Formel (I): 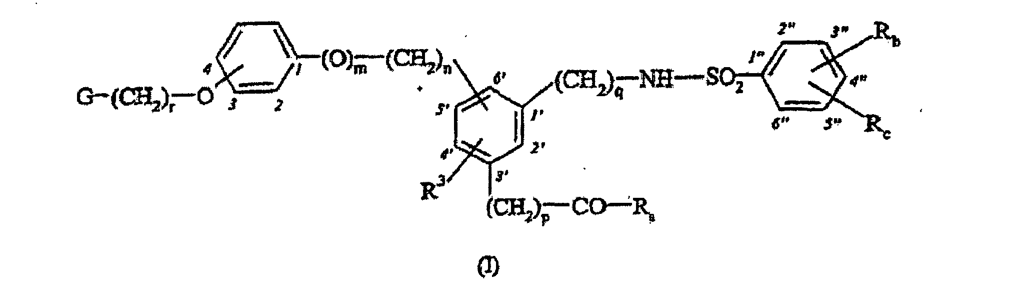 in der
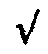 G eine Gruppe der Formel bedeutet: 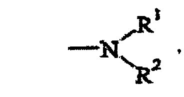 in der R¹ und R² unabhängig voneinander ein Wasserstoffatom, eine Alkyl-, Cycloalkyl-, gegebenenfalls substituierte Aryl-, gegebenenfalls substituierte Arylalkyl-, Cycloalkyl-, Cycloalkylalkyl-, gegebenenfalls substituierte Heteroaryl- oder gegebenenfalls substituierte Heteroarylalkyl-gruppe bedeuten, oder
R¹ und R² zusammen mit dem Stickstoffatom eine Heterocycloalkylgruppe der Formel 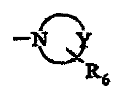 mit 5 bis 7 Kettengliedern bilden, in der Y ein Stickstoffoder Sauerstoffatom oder eine Gruppe CH₂ bedeutet und R₆ ein Wasserstoffatom, eine Alky1-, Cycloalkyl-, Cycloalkylalkyl-, gegebenenfalls substituierte Aryl-, gegebenenfalls substituierte Arylalkyl-, gegebenenfalls substituierte Arylcarbonyl-, gegebenenfalls substituierte Arylcarbonylalkyl-, gegebenenfalls substituierte Diarylalkyl-, gegebenenfalls substituierte Diarylalkenyl-, gegebenenfalls substituierte Heteroaryl-, gegebenenfalls substituierte Heteroarylalkyl-, gegebenenfalls substituierte Heteroarylcarbonyl- oder gegebenenfalls substituierte Heteroarylcarbonylalkyl-gruppe darstellt,
V R³ ein Wasserstoffatom, eine Alkylgruppe oder eine gegebenenfalls substituierte Phenylgruppe bedeutet,
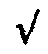 Rₐ eine Hydroxy-, Alkoxy-, gegebenenfalls substituierte Aryloxy-, gegebenenfalls substituierte Arylalkyloxy-, Amino-, Alkylamino-, Dialkylamino-, gegebenenfalls substituierte Arylamino- oder gegebenenfalls substituierte Arylalkylamino-gruppe bedeutet,
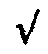 R_{b} und R_{c}, die gleichartig oder verschieden sind, ein Wasserstoffatom, ein Halogenatom, eine Alkylgruppe, eine Alkoxygruppe, eine Hydroxygruppe oder eine Trihalogenalkylgruppe bedeuten,
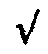 m eine ganze Zahl zwischen 0 und 1 einschließlich bedeutet,
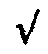 n und q gleichartige oder verschiedenartige ganze Zahlen zwischen 0 und 6 einschließlich bedeuten,
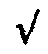 p und r gleichartige oder verschiedenartige ganze Zahlen zwischen 1 und 6 einschließlich bedeuten,
deren Enantiomere und Diastereoisomere sowie deren Additionssalze mit einer pharmazeutisch annehmbaren Säure oder Base, mit der Maßgabe, daß:
- der Begriff Alkyl eine geradkettige oder verzweigte Kette mit 1 bis 6 Kohlenstoffatomen bedeutet,
- der Begriff Alkoxy eine geradkettige oder verzweigte Alkyl-oxy-gruppe bedeutet, die 1 bis 6 Kohlenstoffatome enthält,
- der Begriff Trihalogenalkyl eine Kohlenstoffkette bedeutet, die 1 bis 3 Kohlenstoffatome und 1 bis 3 gleichartige oder verschiedenartige Halogenatome enthält,
- der Begriff Alkenyl eine Kette mit 2 bis 6 Kohlenstoffatomen, die 1 bis 3 Doppelbindungen enthält, bedeutet,
- der Begriff Cycloalkyl eine gesättigte cyclische Gruppe bedeutet, die 3 bis 8 Kohlenstoffatome enthält,
- der Begriff Aryl eine Phenyl- oder Naphthylgruppe bedeutet,
- der Begriff Heteroaryl eine aromatische monocyclische oder bicyclische Gruppe, bei der mindestens einer der Ringe aromatisch ist, bedeutet, die 5 bis 11 Kettenglieder und 1 bis 5 Heteroatome ausgewählt aus Stickstoff, Sauerstoff und Schwefel enthält,
- die Begriffe Diarylalkyl und Diarylalkenyl Alkyl- bzw. Alkenylgruppen bedeuten, wie sie oben definiert worden sind, die durch zwei gleichartige oder verschiedene Arylgruppen, wie sie oben definiert worden sind, substituiert sind,
- der Begriff "substituiert" bezogen auf die Begriffe Phenyl, Aryl, Arylalkyl, Arylcarbonyl, Arylcarbonylalkyl, Diarylalkyl, Diarylalkenyl, Heteroaryl, Heteroarylalkyl, Heteroarylcarbonyl, Heteroarylcarbonylalkyl, Arylamino und Arylalkylamino bedeutet, daß die betreffenden Gruppen am aromatischen Rest durch einen oder zwei gleichartige oder verschiedenartige Substituenten substituiert sind, ausgewählt aus Halogenatomen und Alkyl-, Alkoxy-, Hydroxy-, Cyano-, Nitro-, (gegebenenfalls durch eine oder zwei Alkylgruppen substituierte) Aminogruppen und Gruppen -C(O)R_{d}, worin R_{d} eine Gruppe bedeutet ausgewählt aus Hydroxy, Alkoxy und Amino mit der Maßgabe, daß die Heteroaryl- und Heteroarylalkylgruppen zusätzlich am nicht-aromatischen Rest der Heteroarylgruppe durch eine Oxogruppe substituiert sein können.

2. Verbindungen der Formel (I) nach Anspruch 1, worin p den Wert 2 besitzt, deren Enantiomere, Diastereoisomere sowie deren Additionssalze mit einer pharmazeutisch annehmbaren Säure oder Base.

3. Verbindungen der Formel (I) nach einem der Ansprüche 1 oder 2, worin q den Wert 2 besitzt, deren Enantiomere, Diastereoisomere sowie deren Additionssalze mit einer pharmazeutisch annehmbaren Säure oder Base.

4. Verbindungen der Formel (I) nach einem der Ansprüche 1 bis 3, worin R³ ein Wasserstoffatom bedeutet, deren Enantiomere, Diastereoisomere sowie deren Additionssalze mit einer pharmazeutisch annehmbaren Säure oder Base.

5. Verbindungen der Formel (I) nach einem der Ansprüche 1 bis 4, worin Rₐ eine Hydroxygruppe bedeutet, deren Enantiomere, Diastereoisomere sowie deren Additionssalze mit einer pharmazeutisch annehmbaren Säure oder Base.

6. Verbindungen der Formel (I) nach einem der Ansprüche 1 bis 5, worin R_{b} ein Halogenatom bedeutet, deren Enantiomere, Diastereoisomere sowie deren Additionssalze mit einer pharmazeutisch annehmbaren Säure oder Base.

7. Verbindungen der Formel (I) nach einem der Ansprüche 1 bis 6, worin R_{c} ein Wasserstoffatom bedeutet, deren Enantiomere, Diastereoisomere sowie deren Additionssalze mit einer pharmazeutisch annehmbaren Säure oder Base.

8. Verbindungen der Formel (I) nach einem der Ansprüche 1 bis 7, worin p und q jeweils 2 bedeuten, Rₐ eine Hydroxygruppe bedeutet, R³ und R_{c} jeweils ein Wasserstoffatom bedeuten, R_{b} ein Halogenatom bedeutet und G eine Amino-, Dialkylamino-, Arylalkylamino- oder Heterocycloalkylgruppe der Formel: 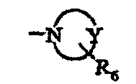 bedeutet, in der Y ein Stickstoffatom oder ein Sauerstoffatom oder eine CH₂-Gruppe darstellt und R₆ aus einem Wasserstoffatom, gegebenenfalls substituierten Arylgruppen und gegebenenfalls substituierten Heteroarylgruppen ausgewählt ist, deren Enantiomere, Diastereoisomere sowie deren Additionssalze mit einer pharmazeutisch annehmbaren Säure oder Base.

9. Verbindungen der Formel (I) nach einem der Ansprüche 1 bis 8, worin G eine Dialkylaminogruppe bedeutet, deren Enantiomere, Diastereoisomere sowie deren Additionssalze mit einer pharmazeutisch annehmbaren Säure oder Base.

10. Verbindungen der Formel (I) nach einem der Ansprüche 1 bis 8, worin G eine substituierte Heterocycloalkylgruppe 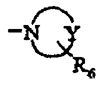 bedeutet, in der:
• 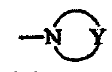 eine Gruppe mit 5 oder 6 Kettengliedern darstellt, wie: eine Pyrrolyl-, Morpholino-, Piperidyl- oder Piperazinyl-gruppe,
• und der Substituent R₆ an einem Kohlenstoff- oder Stickstoffatom des Heterocycloalkylrests ein Wasserstoffatom, einen gegebenenfalls durch ein Halogenatom substituierten Phenylsubstituenten oder eine Heteroarylgruppe mit 9. Kettengliedern, die ein bis zwei Heteroatome ausgewählt aus Stickstoff-, Sauerstoff- und Schwefelatomen umfaßt und gegebenenfalls durch ein Halogenatom substituiert ist, bedeuten,
deren Enantiomere, Diastereoisomere sowie deren Additionssalze mit einer pharmazeutisch annehmbaren Säure oder Base.

11. Verbindungen der Formel (I) nach einem der Ansprüche 1 bis 8 oder 10, bei denen die gegebenenfalls substituierte Heteroarylgruppe eine gegebenenfalls durch ein Halogenatom substituierte Benzisoxazolylgruppe oder eine gegebenenfalls durch ein Halogenatom substituierte Benzisothiazolylgruppe bedeutet, deren Enantiomere. Diastereoisomere sowie deren Additionssalze mit einer pharmazeutisch annehmbaren Säure oder Base.

12. Verbindungen der Formel (I) nach einem der Ansprüche 1 bis 11, worin der Substituent G-(CH₂)ᵣ-O- an eines der beiden Kohlenstoffatome *2* oder *4* der Phenylgruppe gebunden ist, deren Enantiomere, Diastereoisomere sowie deren Additionssalze mit einer pharmazeutisch annehmbaren Säure oder Base.

13. Verbindungen der Formel (I) nach einem der Ansprüche 1 bis 12, worin der Substituent 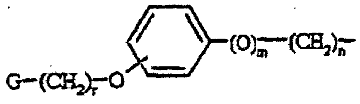 an das Kohlenstoffatom 5' der Phenylgruppe gebunden ist, deren Enantiomere, Diastereoisomere sowie deren Additionssalze mit einer pharmazeutisch annehmbaren Säure oder Base.

14. Verbindungen der Formel (I) nach einem der Ansprüche 1 bis 13, worin R_{b} an das Kohlenstoffatom 4" der Phenylgruppe gebunden ist, deren Enantiomere, Diastereoisomere sowie deren Additionssalze mit einer pharmazeutisch annehmbaren Säure oder Base.

15. Verbindung der Formel (I) nach den Ansprüchen 1 bis 9 und 12 bis 14, nämlich 3-[3-(2-{[(4-Chlorphenyl)-sulfonyl]-amino}-ethyl)-5-(2-{2-[3-(dimethylamlno)-propoxy]-phenyl}-ethyl)-phenyl]-propansäure.

16. Verbindungen der Formel (I) nach den Ansprüchen 1 bis 8, 10 und 12 bis 14, nämlich:
- 3-[3-(2-{[(4-Chlorphenyl)-sulfonyl]-amino}-ethyl)-5-(2-{2-[2-(1-pyrrolidinyl)-ethoxyl-phenyl}-ethyl)-phenyl]-propansäure und
- 3-[3-(2-{[(4-Chlorphenyl)-sulfonyl]-amino}-ethyl)-5-(2-{2-[2-(4-morpholinyl)-ethoxy]-phenyl}-ethyl)-phenyl]-propansäure.

17. Verfahren zur Herstellung der Verbindungen der Formel (I) nach Anspruch 1, **dadurch gekennzeichnet, daß**:
① wenn man die Verbindungen der Formel (I) herstellen will, bei denen m = 0 bedeutet und n von 1 verschieden ist, man als Ausgangsprodukt eine Verbindung der Formel (II) verwendet: 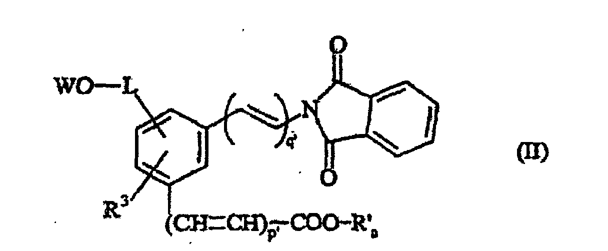 in der R³ die bezüglich der Formel (I) angegebenen Bedeutungen besitzt, R'ₐ eine geradkettige oder verzweigte (C₁-C₆)-Alkylgruppe darstellt, WO-L eine Gruppe der Formel bedeutet: 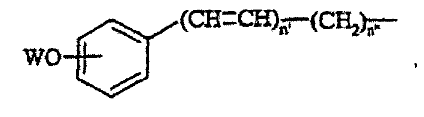 worin W ein Wasserstoffatom oder eine Benzylgruppe darstellt, p' und q' ganze Zahlen zwischen 0 und 3 bedeuten, n' + n" eine ganze Zahl zwischen 0 und 3 bedeuten, wobei, wenn n' 0 bedeutet, n" 0, 2 oder 3 darstellt,
- welche katalytisch reduziert wird, so daß man nach der Debenzylierung, wenn W eine Benzylgruppe darstellt, die Verbindung der Formel (III) erhält: 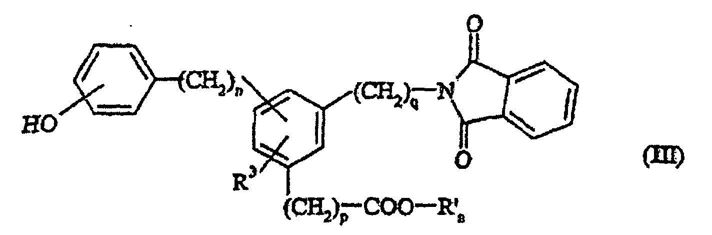 in der R³, n, p und q die bezüglich der Formel (I) angegebenen Bedeutungen besitzen und R'ₐ eine geradkettige oder verzweigte (C₁-C₆)-Alkylgruppe darstellt,
- welche in basischem Medium mit einem Halogenid der Formel kondensiert wird: G-(CH₂)ᵣ-X, in der X ein Halogenatom bedeutet, zur Bildung der Verbindung der Formel (IV): 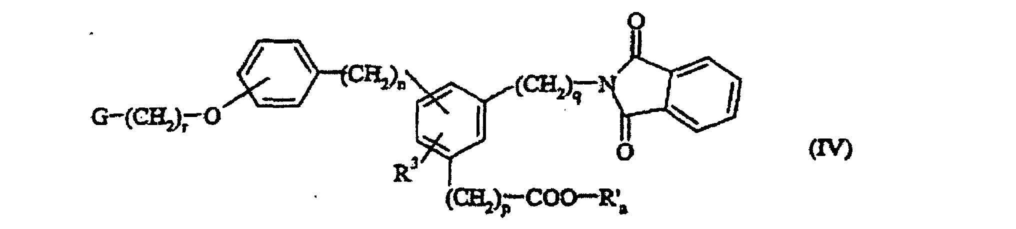 in der G, R³, n, p, q und r die bezüglich der Formel (I) angegebenen Bedeutungen besitzen und R'ₐ eine geradkettige oder verzweigte (C₁-C₆)-Alkylgruppe bedetuet,
- von der die Schutzgruppe durch Abspalten der Phthalimidogruppe in Gegenwart von Hydrazin entfernt wird zur Bildung des entsprechenden Amins, welches seinerseits mit einem gegebenenfalls substituierten Benzolsulfonylchlorid der Formel umgesetzt wird: 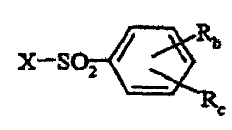 in der R_{b} und R_{c} die bezüglich der Formel (I) angegebenen Bedeutungen besitzen und X ein Halogenatom darstellt,
zur Bildung der Verbindung der Formel (I/a), einem Sonderfall der Verbindungen der Formel (I): 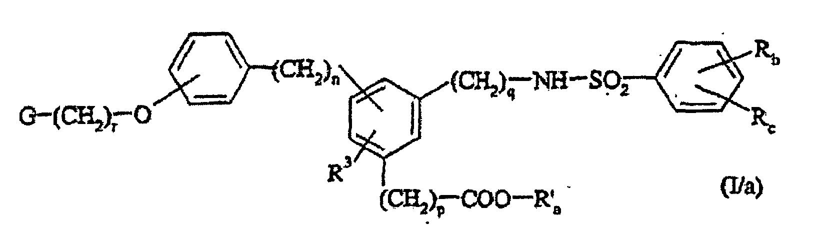 in der G, R_{b}, R_{c}, R³, r, n, p und q die bezüglich der Formel (I) angegebenen Bedeutungen besitzen und R'ₐ eine geradkettige oder verzweigte (C₁-C₆)-Alkylgruppe darstellt,
② wenn man die Verbindungen der Formel (I) herstellen will, bei der m = 1 oder m = 0 und n = 1 bedeuten, man als Ausgangsprodukt eine Verbindung der Formel (II/a) verwendet: 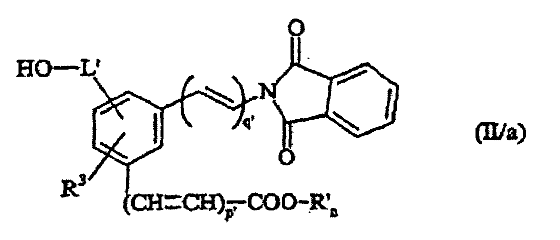 in der R³ die bezüglich der Formel (I) angegebenen Bedeutungen besitzt, R'ₐ eine geradkettige oder verzweigte (C₁-C₆)-Alkylgruppe bedeutet, HO-L' eine Gruppe der Formel darstellt: HO-(CH₂)ₙ-, q' und p' ganze Zahlen zwischen 0 und 3 bedeuten und n eine ganze Zahl zwischen 0 und 6 darstellt,
- welche katalytisch hydriert und halogeniert wird zur Bildung der Verbindung der Formel (V): 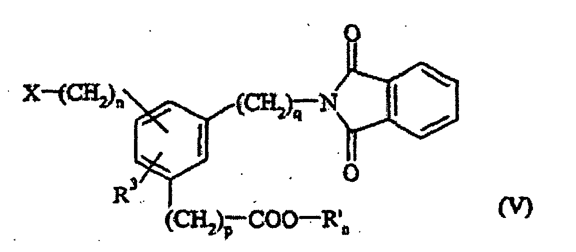 in der R³, n, p und q die bezüglich der Formel (I) angegebenen Bedeutungen besitzen, R'ₐ eine geradkettige oder verzweigte (C₁-C₆)-Alkylgruppe bedeutet und X ein Halogenatom darstellt,
welche Verbindung der Formel (V) man:
- **entweder,** wenn m = 1 bedeutet, einem nucleophilen Angriff mit einer Verbindung der Formel unterworfen wird: 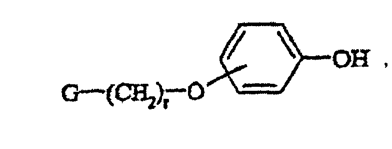 zur Bildung der Verbindung der Formel (VI): 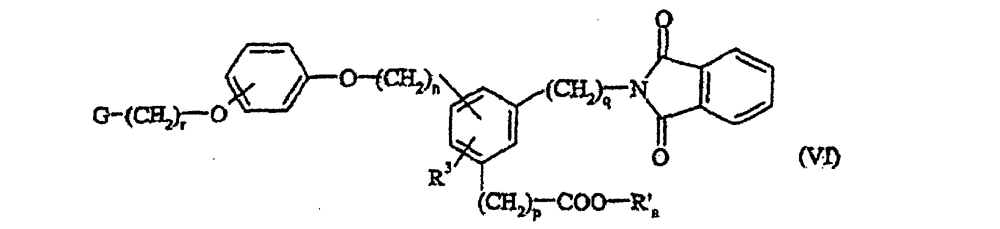 in der G, R³, n, p, q und r die bezüglich der Formel (I) angegebenen Bedeutungen besitzen und R'ₐ eine geradkettige oder verzweigte (C₁-C₆)-Alkylgruppe darstellt,
- von welcher die Schutzgruppe durch Abspalten der Phthalimidogruppe in Gegenwart von Hydrazin entfernt wird zur Bildung des entsprechenden Amins, welches seinerseits mit einem gegebenenfalls substituierten Benzolsulfonylhalogenid der Formel umgesetzt wird: 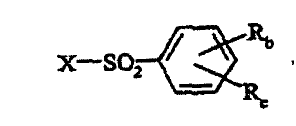 , in der X ein Halogenatom bedeutet, zur Bildung der Verbindung der Formel (I/b), einem Sonderfall der Verbindungen der Formel (I): 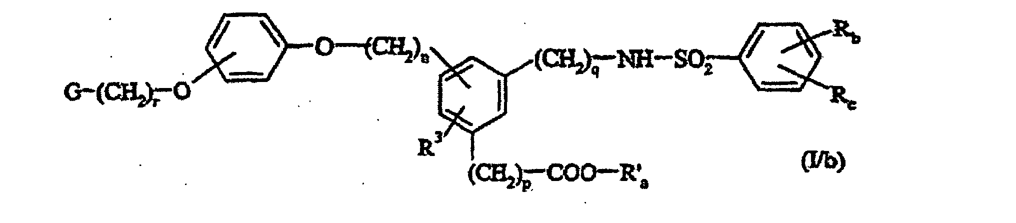 in der G, R_{b}, R_{c}, R³, n, p, q und r die bezüglich der Formel (I) angegebenen Bedeutungen besitzen und R'ₐ eine geradkettige oder verzweigte (C₁-C₆)-Alkylgruppe bedeutet,
- oder. wenn m = 0 und n = 1 bedeuten, der Einwirkung einer Verbindung der Formel 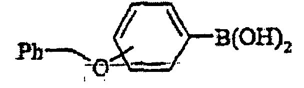 unterworfen wird, so daß man nach der Debenzylierung die Verbindung der Formel (VII) erhält: 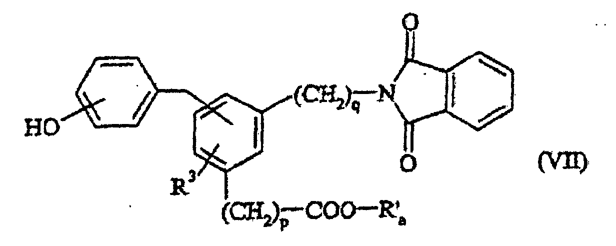 in der R³, p und q die bezüglich der Formel (I) angegebenen Bedeutungen besitzen und R'ₐ eine geradkettige oder verzweigte (C₁-C₆)-Alkylgruppe darstellt,
- welche in basischem Medium mit einem Halogenid der Formel kondensiert wird: G-(CH₂)ᵣ-X, in der X ein Halogenatom bedeutet, zur Bildung der Verbindung der Formel (VIII): 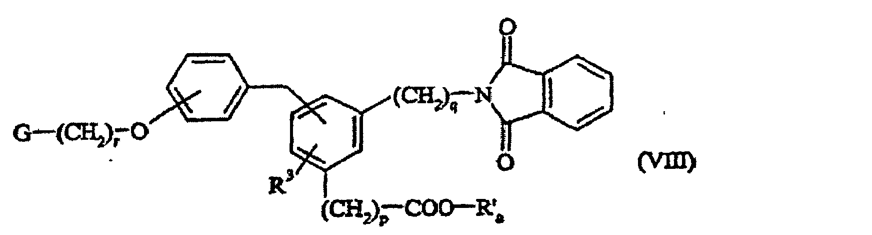 in der G, R³, p, q und r die bezüglich der Formel (I) angegebenen Bedeutungen besitzen und R'ₐ eine geradkettige oder verzweigte (C₁-C₆)-Alkylgruppe darstellt,
- von der die Schutzgruppe durch Abspalten der Phthalimidogruppe in Gegenwart von Hydrazin entfernt wird zur Bildung des entsprechenden Amins, welches seinerseits mit einem gegebenenfalls substituierten Benzolsulfonylhalogenid der Formel umgesetzt wird: 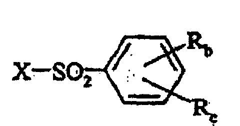 zur Bildung der Verbindung der Formel (I/c), einem Sonderfall der Verbindungen der Formel (I): 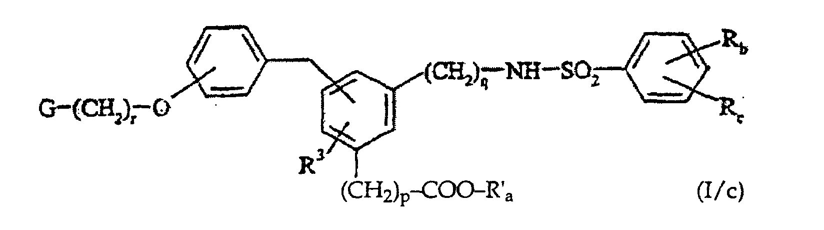 in der G, R_{b}, R_{c}, R³, p, q und r die bezüglich der Formel (I) angegebenen Bedeutungen besitzen und R'ₐ eine geradkettige oder verzweigte (C₁-C₆)-Alkylgruppe darstellt,
welche Verbindungen der Formeln (I/a), (I/b) oder (I/c) einer Hydrolyse der Esterfunktion in basischem oder saurem Medium in Abhängigkeit von den reaktiven Gruppen, die an dem Molekül vorhanden sind, unterworfen werden können zur Bildung der Verbindung der Formel (I/d): 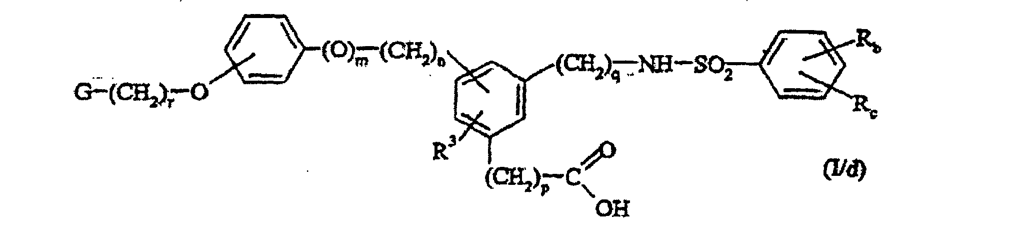 einem Sonderfall der Verbindungen der Formel (I), worin G, R_{b}, R_{c}, R³, m, n, p, q und r die bezüglich der Formel (I) angegebenen Bedeutungen besitzen,
welche Verbindungen der Formeln (I/a), (I/b), (I/c) und (I/d), welche die Gesamtheit der Verbindungen der Formel (I) bilden:
- gegebenenfalls mit Hilfe einer klassischen Reinigungsmethode gereinigt werden können,
- gegebenenfalls mit Hilfe einer klassischen Trennmethode in ihre Stereoisomeren aufgetrennt werden können, und
- man gewünschtenfalls in ihre Additionssalze mit einer pharmazeutisch annehmbaren Säure oder Base überführt,
wobei es sich versteht, daß man zu einem beliebigen geeigneten Zeitpunkt des oben beschriebenen Verfahrens die Carbonsäurefunktion verestern kann oder die Carbonsäureesterfunktion zu der entsprechenden Säure hydrolysieren kann, wobei die letztere erneut zum Zwecke der Synthese in einen anderen Ester überführt werden kann.

18. Pharmazeutische Zubereitungen enthaltend als Wirkstoff mindestens eine Verbindung nach einem der Ansprüche 1 bis 16 allein oder in Kombination mit einem oder mehreren inerten, nichttoxischen, pharmazeutisch annehmbaren Trägermaterialien oder Bindemitteln.

19. Pharmazeutische Zubereitungen nach Anspruch 18, enthaltend mindestens einen Wirkstoff nach einem der Ansprüche 1 bis 16 für die Herstellung von Arzneimitteln, die nützlich sind als Antagonisten der Rezeptoren von TXA₂ und der Rezeptoren 5-HT₂.

20. Pharmazeutische Zubereitungen nach Anspruch 18, enthaltend mindestens einen Wirkstoff nach einem der Ansprüche 1 bis 16 für die Herstellung von Arzneimitteln, die nützlich sind bei der Behandlung von atherothrombotischen kardiovaskulären Erkrankungen, wie Myokardinfarkt, Angina pectoris. Schlaganfälle, der Raynaudschen Krankheit oder Asthma, Bronchospasmen, Migräne und Venenerkrankungen.

## Claims

1. Compounds of formula (I) : 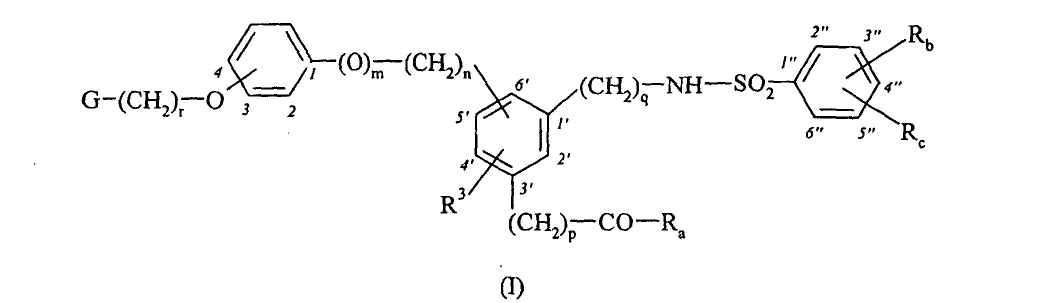 wherein :
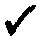 G represents a group such as : 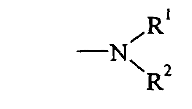 wherein R¹ and R² independently represent a hydrogen atom or an alkyl, cycloalkyl, optionally substituted aryl, optionally substituted arylalkyl, cycloalkyl, cycloalkylalkyl, optionally substituted heteroaryl or optionally substituted heteroarylalkyl group,
or,
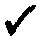 R¹ and R², together with the nitrogen atom, form a heterocycloalkyl group of formula 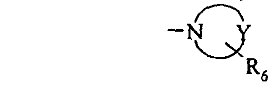 having from 5 to 7 ring members wherein Y represents a nitrogen atom, an oxygen atom or a CH₂ group and R₆ represents a hydrogen atom or an alkyl, cycloalkyl, cycloalkylalkyl, optionally substituted aryl, optionally substituted arylalkyl, optionally substituted arylcarbonyl, optionally substituted arylcarbonylalkyl, optionally substituted diarylalkyl, optionally substituted diarylalkenyl, optionally substituted heteroaryl, optionally substituted heteroarylalkyl, optionally substituted heteroarylcarbonyl or optionally substituted heteroarylcarbonylalkyl group,
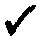 R³ represents a hydrogen atom, an alkyl group or an optionally substituted phenyl group,
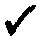 Rₐ represents a hydroxy, alkoxy, optionally substituted aryloxy, optionally substituted arylalkyloxy, amino, alkylamino, dialkylamino, optionally substituted arylamino or optionally substituted arylalkylamino group,
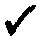 R_{b} and R_{c}, which may be identical or different, represent a hydrogen atom, a halogen atom, an alkyl group, an alkoxy group, a hydroxy group or a trihaloalkyl group,
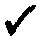 m is an integer 0 or 1,
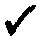 n and q are identical or different integers of from 0 to 6 inclusive,
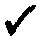 p and r are identical or different integers of from 1 to 6 inclusive,
their enantiomers, diastereoisomers and also addition salts thereof with a pharmaceutically acceptable acid or base,
wherein :
- the term "alkyl" denotes a linear or branched chain having from 1 to 6 carbon atoms,
- the term "alkoxy" denotes a linear or branched alkyl-oxy group containing from 1 to 6 carbon atoms,
- the term "trihaloalkyl" denotes a carbon chain containing from 1 to 3 carbon atoms and from 1 to 3 identical or different halogen atoms,
- the term "alkenyl" denotes a chain having from 2 to 6 carbon atoms and containing from 1 to 3 double bonds,
- the term "cycloalkyl" denotes a saturated cyclic group containing from 3 to 8 carbon atoms,
- the term "aryl" denotes a phenyl or naphthyl group,
- the term "heteroaryl" denotes an aromatic monocyclic group or a bicyclic group in which at least one of the rings is aromatic, having from 5 to 11 ring members and from 1 to 5 hetero atoms selected from nitrogen, oxygen and sulphur,
- the terms "diarylalkyl" and "diarylalkenyl" denote, respectively, alkyl and alkenyl groups as defined hereinbefore, substituted by two identical or different aryl groups as defined hereinbefore,
- the term "substituted" relating to phenyl, aryl, arylalkyl, arylcarbonyl, arylcarbonylalkyl, diarylalkyl, diarylalkenyl, heteroaryl, heteroarylalkyl, heteroarylcarbonyl, heteroarylcarbonylalkyl, arylamino and arylalkylamino denotes that the groups in question are substituted on the aromatic moiety by one or two identical or different substituents selected from the halogen atoms and the groups alkyl, alkoxy, hydroxy, cyano, nitro, amino (optionally substituted by one or two alkyl groups) and -C(O)R_{d}, R_{d} representing a group selected from hydroxy, alkoxy and amino, wherein the heteroaryl and heteroarylalkyl groups may in addition be substituted by an oxo group on the non-aromatic moiety of the heteroaryl.

2. Compounds of formula (I) according to claim 1 wherein p is 2, their enantiomers, diastereoisomers and also addition salts thereof with a pharmaceutically acceptable acid or base.

3. Compounds of formula (I) according to claim 1 or 2 wherein q is 2, their enantiomers, diastereoisomers and also addition salts thereof with a pharmaceutically acceptable acid or base.

4. Compounds of formula (I) according to any one of claims 1 to 3 wherein R³ represents a hydrogen atom, their enantiomers, diastereoisomers and also addition salts thereof with a pharmaceutically acceptable acid or base.

5. Compounds of formula (I) according to any one of claims 1 to 4 wherein Rₐ represents a hydroxy group, their enantiomers, diastereoisomers and also addition salts thereof with a pharmaceutically acceptable acid or base.

6. Compounds of formula (I) according to any one of claims 1 to 5 wherein R_{b} represents a halogen atom, their enantiomers, diastereoisomers and also addition salts thereof with a pharmaceutically acceptable acid or base.

7. Compounds of formula (I) according to any one of claims 1 to 6 wherein R_{c} represents a hydrogen atom, their enantiomers, diastereoisomers and also addition salts thereof with a pharmaceutically acceptable acid or base.

8. Compounds of formula (I) according to any one of claims 1 to 7 wherein p and q are each 2, Rₐ represents a hydroxy group, R³ and R_{c} each represents a hydrogen atom, R_{b} represents a halogen atom and G represents an amino, dialkylamino or arylalkylamino group, or a heterocycloalkyl group of formula: 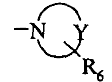 wherein Y represents a nitrogen atom, an oxygen atom or a CH₂ group and R₆ is selected from a hydrogen atom and the groups optionally substituted aryl and optionally substituted heteroaryl, their enantiomers, diastereoisomers and also addition salts thereof with a pharmaceutically acceptable acid or base.

9. Compounds of formula (I) according to any one of claims 1 to 8 wherein G represents a dialkylamino group, their enantiomers, diastereoisomers and also addition salts thereof with a pharmaceutically acceptable acid or base.

10. Compounds of formula (I) according to any one of claims 1 to 8 wherein G represents a substituted heterocycloalkyl group 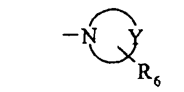 wherein :
• 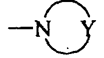 represents a group having 5 or 6 ring members such as : pyrrolyl, morpholino, piperidyl or piperazinyl,
• and R₆ substituted on a carbon or nitrogen atom of the heterocycloalkyl represents a hydrogen atom, a phenyl substituent optionally substituted by a halogen atom, or a heteroaryl group having 9 ring members containing one or two hetero atoms selected from nitrogen, oxygen and sulphur and optionally substituted by a halogen atom,
their enantiomers, diastereoisomers and also addition salts thereof with a pharmaceutically acceptable acid or base.

11. Compounds of formula (I) according to any one of claims 1 to 8 or 10 wherein the optionally substituted heteroaryl group is a benzisoxazolyl group optionally substituted by a halogen atom, or a benzisothiazolyl group optionally substituted by a halogen atom, their enantiomers, diastereoisomers and also addition salts thereof with a pharmaceutically acceptable acid or base.

12. Compounds of formula (I) according to any one of claims 1 to 11 wherein the substituent G-(CH₂)ᵣ-O- is bonded to one of the two carbon atoms *2* or *4* of the phenyl group, their enantiomers, diastereoisomers and also addition salts thereof with a pharmaceutically acceptable acid or base.

13. Compounds of formula (I) according to any one of claims 1 to 12 wherein the substituer 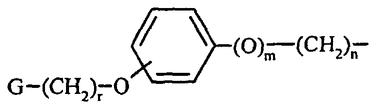 is bonded to the 5' carbon atom of the phenyl group, their enantiomers, diastereoisomers and also addition salts thereof with a pharmaceutically acceptable acid or base.

14. Compounds of formula (I) according to any one of claims 1 to 13 wherein R_{b} is bonded to the 4" carbon atom of the phenyl group, their enantiomers, diastereoisomers and also addition salts thereof with a pharmaceutically acceptable acid or base.

15. Compound of formula (I) according to claims 1 to 9 and 12 to 14 which is 3-[3-(2-{[(4-chlorophenyl)sulphonyl]amino}ethyl)-5-(2-{2-[3-(dimethylamino)propoxy]-phenyl}ethyl)phenyl]propanoic acid.

16. Compounds of formula (I) according to claims 1 to 8, 10 and 12 to 14 which are :
- 3-[3-(2-{[(4-chlorophenyl)sulphonyl]amino}ethyl)-5-(2-{2-[2-(1-pyrrolidinyl)ethoxy]phenyl}ethyl)phenyl]propanoic acid,
- and 3-[3-(2-{[(4-chlorophenyl)sulphonyl]amino}ethyl)-5-(2-{2-[2-(4-morpholinyl)ethoxy]phenyl}ethyl)phenyl]propanoic acid.

17. Process for the preparation of compounds of formula (I) according to claim 1, **characterised in that** :
1) when it is desired to obtain compounds of formula (I) wherein m=0 and n is other than 1, there is used as starting material a compound of formula (II) : 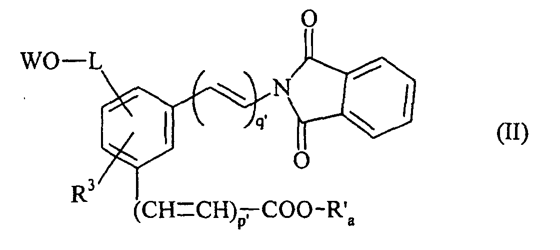 wherein R³ is as defined for formula (I), R'ₐ represents a linear or branched (C₁-C₆)alkyl group, WO-L represents a group of formula: 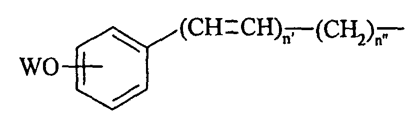 wherein W represents a hydrogen atom or a benzyl group, p' and q' are integers from 0 to 3, n'+n" = an integer of from 0 to 3 wherein when n' is 0 n" is 0, 2 or 3,
- which is catalytically reduced to yield, after debenzylation when W represents a benzyl group, a compound of formula (III) : 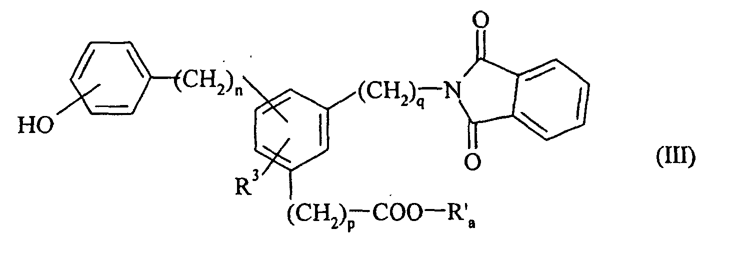 wherein R³, n, p and q are as defined for formula (I) and R'ₐ represents a linear or branched (C₁-C₆)alkyl group,
- which is condensed in basic medium with a halide of formula : G-(CH₂)ᵣ-X, wherein X represents a halogen atom, to yield a compound of formula (IV) : 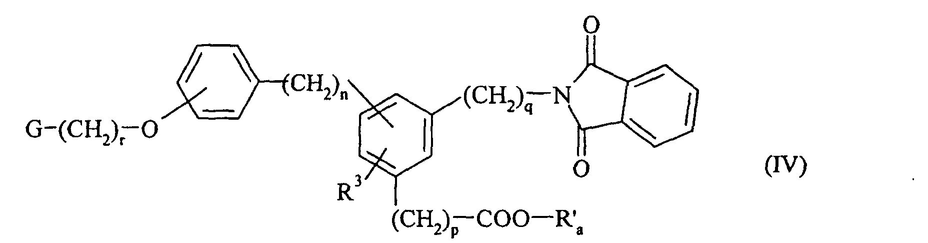 wherein G, R³, n, p, q and r are as defined for formula (I) and R'ₐ represents a linear or branched (C₁-C₆)alkyl group,
- which is deprotected by cleavage of the phthalimido group in the presence of hydrazine to yield the corresponding amine which, in turn, reacts with an optionally substituted benzenesulphonyl halide of formula : 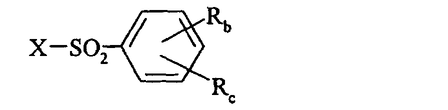 wherein R_{b} and R_{c} are as defined for formula (I) and X represents a halogen atom, to yield a compound of formula (I/a), a particular case of the compounds (I) : 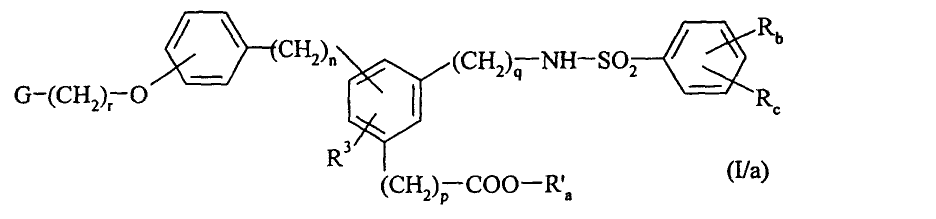
wherein G, R_{b}, R_{c}, R³, r, n, p and q are as defined for formula (I) and R'ₐ represents a linear or branched (C₁-C₆)alkyl group,
2) when it is desired to obtain compounds of formula (I) wherein m=1, or wherein m=0 and n=1, there is used as starting material a compound of formula (II/a) : 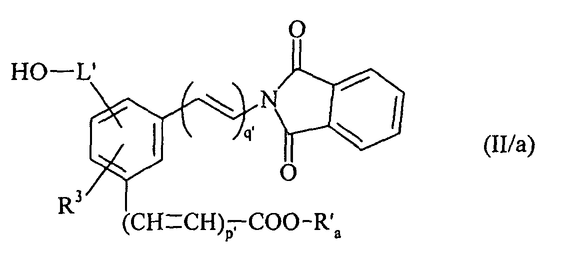 wherein R³ is as defined for formula (I), R'ₐ represents a linear or branched (C₁-C₆)alkyl group, HO-L' represents a group of formula: HO-(CH₂)ₙ-, q', p' are integers of from 0 to 3 and n is an integer of from 0 to 6,
- which is catalytically reduced and halogenated to yield a compound (V) : 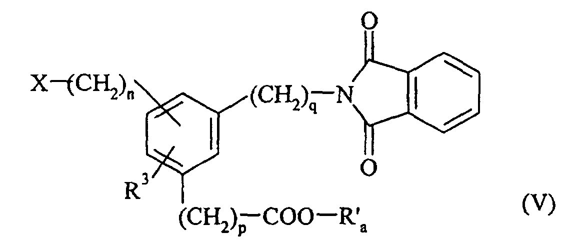 wherein R³, n, p and q are as defined for formula (I), R'ₐ represents a linear or branched (C₁-C₆)alkyl group and X represents a halogen atom,
which compound of formula (V):
- **either**, when m = 1, is subjected to the nucleophilic attack of a compound of formula: 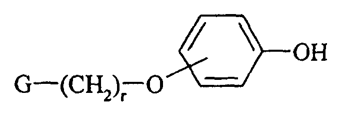 to yield a compound of formula (VI) : 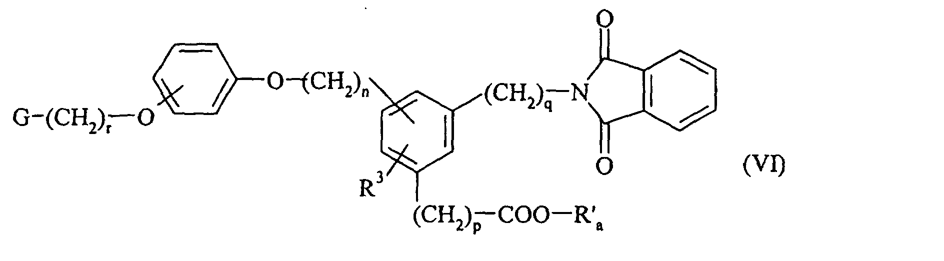 wherein G, R³, n, p, q and r are as defined for formula (I) and R'ₐ represents a linear or branched (C₁-C₆)alkyl group,
- which is deprotected by cleavage of the phthalimido group in the presence of hydrazine to yield the corresponding amine which, in turn, reacts with an optionally substituted benzenesulphonyl halide of formula: 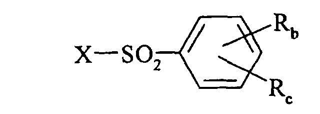 wherein X represents a halogen atom, to yield a compound of formula (I/b), a particular case of the compounds of formula (I) : 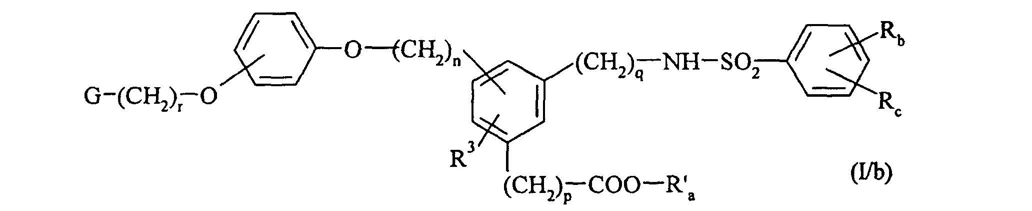 wherein G, R_{b}, R_{c}, R³, n, p, q and r are as defined for formula (I) and R'ₐ represents a linear or branched (C₁-C₆)alkyl group,
- **or**, when m = 0 and n = 1, is subjected to the action of a compound of formula 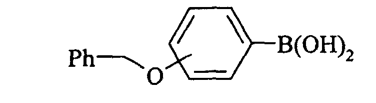 to yield, after debenzylation, a compound of formula (VII) : 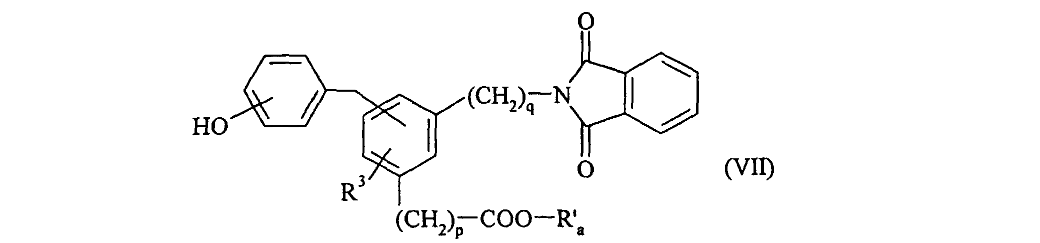 wherein R³, p and q are as defined for formula (I) and R'ₐ represents a linear or branched (C₁-C₆)alkyl group,
- which is condensed in basic medium with a halide of formula G-(CH₂)ᵣ-X, wherein X represents a halogen atom, to yield a compound of formula (VIII) : 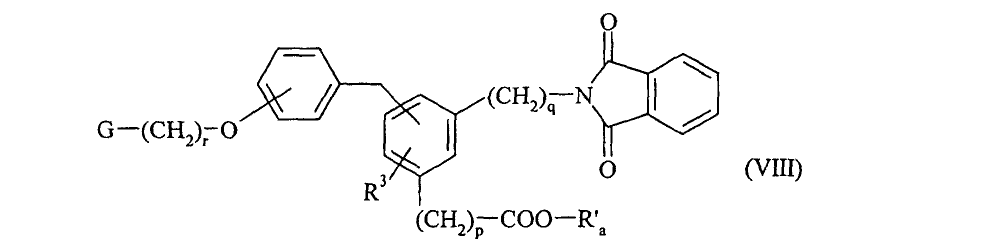 wherein G, R³, p, q and r are as defined for formula (I) and R'ₐ represents a linear or branched (C₁-C₆)alkyl group, ,
- which is deprotected by cleavage of the phthalimido group in the presence of hydrazine to yield the corresponding amine which, in turn, reacts with an optionally substituted benzenesulphonyl halide of formula : 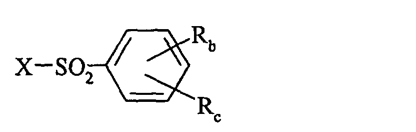 to yield a compound of formula (1/c), a particular case of the compounds (1) : 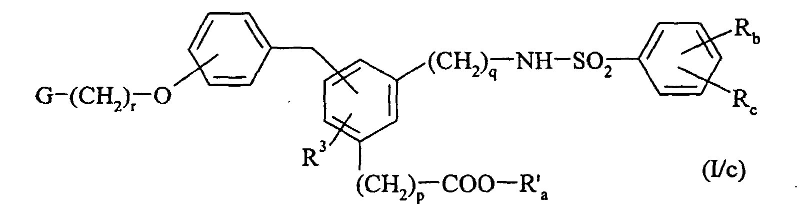 wherein G, R_{b}, R_{c}, R³, p, q and r are as defined for formula (I) and R'ₐ represents a linear or branched (C₁-C₆)alkyl group,
which compounds of formula (I/a), (I/b) or (I/c) may be subjected to hydrolysis of the ester function, in basic or acidic medium according to the reactive groups present in the molecule, to yield a compound of formula (I/d) : 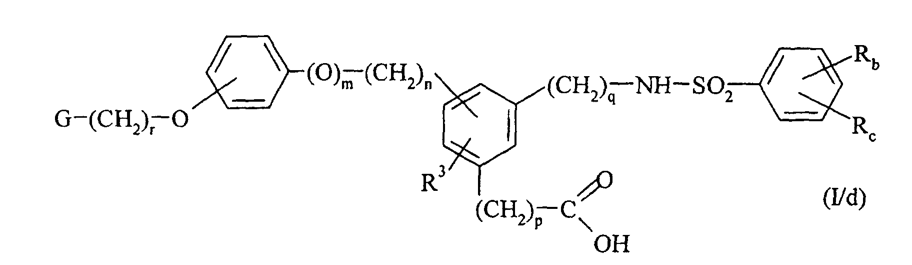 a particular case of the compounds of formula (I) wherein G, R_{b}, R_{c}, R³, m, n, p, q and r are as defined for formula (I),
which compounds (I/a), (I/b), (I/c) and (I/d), constituting the totality of the compounds of formula (I), :
- may, if desired, be purified according to a conventional purification technique,
- are optionally separated into the stereoisomers according to a conventional separation technique,
- are, if desired, converted into their additions salts with a pharmaceutically acceptable acid or base,
it being understood that at any moment considered appropriate during the course of the process described above, the carboxylic acid function may be esterified or the carboxylic ester function may be hydrolysed to the corresponding acid, it being possible for the latter to be converted again to another ester as required by the synthesis.

18. Pharmaceutical compositions comprising as active ingredient at least one compound according to any one of claims 1 to 16 alone or in combination with one or more pharmaceutically acceptable, inert, non-toxic excipients or carriers.

19. Pharmaceutical compositions according to claim 18 comprising at least one active ingredient according to any one of claims 1 to 16 for use in the manufacture of medicaments useful as antagonists of TXA₂ receptors and 5-HT₂ receptors.

20. Pharmaceutical compositions according to claim 18 comprising at least one active ingredient according to any one of, claims 1 to 16 for use in the manufacture of medicaments useful in the treatment of atherothrombotic cardiovascular diseases, such as myocardial infarction, angina pectoris, cerebral vascular accidents, Raynaud's disease, or also asthma, bronchospasms, migraine and venous diseases.
